# EUROPEAN PATENT APPLICATION

(11) **EP 4 046 651 A1**
(43) Date of publication of application: **24.08.2022**
(21) Application number: 21158753.0
(22) Date of filing: 23.02.2021
(51) Int. Cl.: A61K 39/00, C12Q 1/6886

(54) **METHOD FOR PRODUCING AN MRNA TUMOR VACCINE**

(71) Applicant: baseclick GmbH, 82061 Neuried (DE)
(72) Inventor: FRISCHMUTH, Thomas, 22587 Hamburg (DE); SOBOTTA, Jessica, 80336 München (DE); CROCE, Stefano, 81669 München (DE); KIERSZNIOWSKA, Sylwia, 14476 Potsdam (DE); CAMPANÓN, Luis Montiel, 81369 München (DE)
(74) Representative: Böhm, Brigitte

(57) **Abstract**

The inventive method for producing an mRNA-tumor vaccine for a patient suffering from a tumor comprises the steps of: i) identifying by genetic analysis specific mRNA transcripts, which are produced in a tumor cell of said patient and/or tumor-specific peptides or proteins encoded by said tumor-specific mRNA transcripts, ii) producing a first set of synthetic mRNA molecules corresponding to the specific mRNA transcripts and/or encoding the tumor-specific peptides or proteins identified in step i), or portions thereof; iii) *in vitro* transfecting immune cells with said first set of synthetic mRNA molecules produced in step ii) encoding said tumor-specific peptides, proteins or portions thereof and expressing said tumor-specific peptides, proteins or portions thereof in said transfected immune cells; iv) identifying tumor-specific peptides, proteins or portions thereof which are immunogenic, particularly which are presented by MHC (class I or class II) molecules of said immune cells; v) producing a set of synthetic mRNA molecules encoding immunogenic tumor-specific peptides or proteins identified in step iv) or portions thereof or peptides or proteins which include such immunogenic tumor-specific peptides or proteins or portions thereof; vi) *in vitro* priming T cells with said set of synthetic mRNA molecules produced in step v) encoding said immunogenic tumor-specific peptides, proteins or portions thereof; vii) identifying at least one peptide, protein or part thereof which triggers and/or increases an immune response in said T-cells in step vi); and viii) producing at least one synthetic mRNA molecule encoding a peptide, protein or portion thereof identified in step vii) for use as a personalized tumor vaccine in said patient. Such personalized tumor vaccine is a further aspect of the present invention.

## Description

The present invention relates to the field of nucleic acid-based vaccination. In particular, the present invention relates to methods for producing personalized mRNA tumor vaccines for patients suffering from a tumor disease, to mRNA molecules which encode peptides which are specifically expressed by a tumor of a patient and which elicit an immune response upon administration to the patient, to pharmaceutical compositions and combinations comprising such mRNA molecule as a tumor vaccine and to methods of treating a cancer disease or to inhibit growth and/or (re-)development of a tumor in a patient.

### Background of the invention

Cancer is one of the major causes for death especially in industrialized nations. For the medical treatment of cancer patients, a variety of different medical approaches has been developed. Chemotherapy, radiotherapy and combinations of the two have contributed to treatment of the patients which very often also includes surgical intervention and all these methods have been improved and refined over the last decades to become more successful. Nevertheless, depending on the type of cancer a patient is suffering from and the disease stage at which the disease is detected and treatment initiated, only a limited number of patients can be cured or long lasting remissions be achieved.

More recently, cancer immune therapy has become a focus of interest. Immune therapy is based on an activation of the immune system. The activation of the immune system of a patient suffering from a tumor leads to a response in which so-called immune effector cells work together to defend the body against the tumor by recognizing and attacking antigens which are specifically expressed on the surface of tumor cells.

Immune therapy is especially valuable in the context of personalized medicine. While many current approaches in cancer treatment rely on a "one-size-fits-all" strategy, or while stratified medicine warrants a tailored medicinal care only to a group or subgroup of patients with a known disease, precision medicine ensures delivery of the right personalized medicinal care to the right patient at the right time. Precision medicine aims to maximize the probability of curing a disease or ailment whilst minimizing the potential side effect of medicinal interventions in individual patients.

New technologies today enable a rapid analysis of total genomes and transcriptomes (genomics and transcriptomics), protein variations (proteomics) and metabolite composition (metabolomics). Data integration (omics) including environment, nutrition and diseases (metadata) leads to individualized health/disease characterization (phenomics). The new technologies have led to new diagnostic tools and approaches. Accumulation of data and development of algorithms (Al) for data analysis allow for a meaningful patient stratification.

Drug development currently concentrates on group (cluster) treatment, whereby drugs on the market expand to other diseases or dosing/treatment regimes. New drug classes for precision medicine include siRNA, mRNA, DNA etc.

Research in the field of cancer treatment has also led to proposals for using vaccines based on either delivery of peptides corresponding to specific tumor markers or mRNA encoding such peptides. Nevertheless, there is still a need for improved therapeutic methods and for the provision of highly specific molecules, which are able to make a tremendous difference in treating patients suffering from all different kinds of cancer diseases. The present invention aims at solving such object and providing highly specific personalized vaccines for individual patients. The invention further aims at providing mRNA tumor vaccines, which are specifically designed to treat cancer diseases in a certain individual and to pharmaceutical compositions comprising such mRNA for use in cancer immune therapy.

### Summary of the Invention

The present invention solves the above-mentioned objects by providing an improved method for producing an effective and specific mRNA tumor vaccine for a patient suffering from a tumor. The invention further gives access to mRNA molecules, which encode a peptide, protein or portion thereof, which is specifically expressed in a tumor of a patient and which peptide, protein or portion thereof elicits an immune response directed against such tumor in the patient.

In a first aspect, the invention relates to a method for producing an mRNA tumor vaccine for a patient suffering from a tumor, which method comprises the following steps:
i) identifying by genetic analysis specific mRNA transcripts, which are produced in a tumor cell of said patient and/or tumor-specific peptides or proteins encoded by said tumor-specific mRNA transcripts,
ii) producing a first set of synthetic mRNA molecules corresponding to the specific mRNA transcripts and/or encoding the tumor-specific peptides or proteins identified in step i), or portions thereof;
iii) *in vitro* transfecting immune cells with said first set of synthetic mRNA molecules produced in step ii) encoding said tumor-specific peptides, proteins or portions thereof and expressing said tumor-specific peptides, proteins or portions thereof in said transfected immune cells;
iv) identifying tumor-specific peptides, proteins or portions thereof which are immunogenic, particularly which are presented by MHC (class I or class II) molecules of said immune cells;
v) producing a set of synthetic mRNA molecules encoding immunogenic tumor-specific peptides or proteins identified in step iv) or portions thereof or peptides or proteins which include such immunogenic tumor-specific peptides or proteins or portions thereof;
vi) *in vitro* priming T cells with said set of synthetic mRNA molecules produced in step v);
vii) identifying at least one peptide, protein or part thereof which triggers and/or increases an immune response in said T-cells in step vi); and
viii) producing at least one synthetic mRNA molecule encoding a peptide, protein or portion thereof identified in step vii) for use as a personalized tumor vaccine in said patient.

In a second aspect, the invention relates to an mRNA molecule, which encodes within its ORF a peptide, protein or portion thereof, which is specifically expressed in a tumor of a patient, but not in healthy cells, and which peptide, protein or portion thereof upon expression in immune cells of said patient elicits an immune response directed against such tumor.

A third and fourth aspect of the invention relates to a pharmaceutical composition and a pharmaceutical combination, which are a cancer vaccine and comprise an mRNA molecule, which encodes a peptide, protein or portion thereof, which elicits an immune response directed against a tumor of a patient.

In a fifth aspect, the invention is directed to a method for treating a cancer disease or inhibit growth and/or (re-)development of a tumor in a patient by producing an mRNA tumor vaccine, which is specific for said cancer or tumor of said patient, by
i) identifying by genetic analysis specific mRNA transcripts, which are produced in a tumor cell of said patient and/or tumor-specific peptides or proteins encoded by said tumor-specific mRNA transcripts,
ii) producing a first set of synthetic mRNA molecules corresponding to the specific mRNA transcripts and/or encoding the tumor-specific peptides or proteins identified in step i), or portions thereof;
iii) in vitro transfecting immune cells with said first set of synthetic mRNA molecules produced in step ii) encoding said tumor-specific peptides, proteins or portions thereof and expressing said tumor-specific peptides, proteins or portions thereof in said transfected immune cells;
iv) identifying tumor-specific peptides, proteins or portions thereof which are immunogenic, particularly which are presented by MHC (class I or class II) molecules of said immune cells;
v) producing a set of synthetic mRNA molecules encoding immunogenic tumor-specific peptides or proteins identified in step iv) or portions thereof or peptides or proteins which include such immunogenic tumor-specific peptides or proteins or portions thereof;
vi) *in vitro* priming T cells with said set of synthetic mRNA molecules produced in step v) encoding said immunogenic tumor-specific peptides, proteins or portions thereof;
vii) identifying at least one peptide, protein or part thereof which triggers and/or increases an immune response in said T-cells in step vi);
viii) producing at least one synthetic mRNA molecule encoding a peptide, protein or portion thereof identified in step vii) for use as a personalized mRNA tumor vaccine in said patient;
and administering an effective amount of said mRNA tumor vaccine to said patient.

### Detailed Description of the Invention and of Preferred Embodiments

The present invention relates to mRNA vaccine technologies and describes an approach, which results in the production of highly efficient and personalized vaccines, which provoke a very specific immune response against tumor cells in a patient. The inventive mRNA tumor vaccine can be used without a risk of the vaccination resulting in further tumor growth or causing new cancers, since mRNA, which is administered to a patient, will not be included into the genome of a patient, but rather will be degraded after a certain time by the body's own mechanisms. Accordingly, the administration of an mRNA vaccine has a transient effect only, and cannot induce permanent mutations. Despite this temporally limited availability of the vaccine mRNA in the patient's body, the translation into the tumor-specific peptide(s), protein(s) or portion(s) thereof is effective and the response of the immune system leads to antibody as well as memory cell production.

The combination of process steps of the inventive method for producing a personalized mRNA-tumor vaccine, further aspects and preferred embodiments of the invention is explained in more detail in the following:
The inventive method includes a first step i) which leads to the identification of specific mRNA transcripts which are produced in a tumor cell of a patient for whom a personalized vaccine is to be prepared. Such transcripts include tumor-specific peptides or proteins. The identification of specific mRNA transcripts can be performed by genetic analysis according to known methods, e.g. using next generation sequencing methods which enable the determination of complete transcriptome or antigen profiles which are specific for a patient. Comparison of antigen profiles of cancer cells vs. non-cancer cells of the same patient or vs. cells of a healthy person, or other similar approaches can be employed for the detection and selection of tumor-specific antigens which can be considered as candidates for an anti-cancer vaccination. The tumor or the cancer disease for which a specific vaccine is provided by the method of the present invention can be any kind of cancer or tumor of any organ or tissue, including solid tumors. Also circulating tumor cells and metastatic offshoots can be the target of an immunization treatment and, thus, be used as a sample for detecting tumor specific mutations in such tumorous cells.

Amongst the tumor specific mRNA transcripts of such patient, there can be encoded peptides or proteins which have already been detected in other patients suffering e.g. from the same tumor and being affected by the same genetic mutation. mRNA vaccines encoding such tumor-type specific peptides or proteins can of course be included, e.g. in a vaccination cocktail comprising several types of mRNA vaccines. However, the present invention especially aims at detecting patient-specific transcription patterns and providing a vaccination against such patient-specific tumor associated peptides and/or proteins or portions thereof with maximum effect.

Samples to be used for identifying tumor specific mRNA transcripts, peptides and proteins can be of any type, especially biopsies of solid tumors or blood samples in which tumor cells are included.

In step ii) of the inventive method, the results of the genetic analysis of step i) are utilized to produce a first set of synthetic mRNA molecules. This first set of mRNA molecules corresponds to detected tumor-specific mRNA transcripts and/or encodes detected tumor-specific peptides, proteins or portions thereof. As it is common knowledge that cancer cells can include a large number of mutations on the genomic level, also a multitude of tumor-specific mRNA transcripts might be detected in the analysis of step i). Accordingly, the first set of synthetic mRNA molecules can include a rather high number of different mRNAs. While it is possible and can be advantageous in some situations to include about 5 or more different tumor-specific mRNA molecules in the first set of mRNA molecules, it is usually preferred to include at least 50 mRNA molecules and more, or even higher numbers. The number of mRNAs can be adjusted according to the complexity of the situation and the available analytical resources.

The synthetic mRNA molecules can be designed as considered appropriate to encode tumor-specific peptides, proteins or selected portions or epitopes thereof and to elicit an immune response against these molecules. The synthetic mRNA molecules include sequences which encode peptides or proteins or portions thereof featuring the detected tumor-specific mutation(s), however, they can also contain further sequences which facilitate or promote presentation of the encoded peptides or proteins or epitopes thereof by the major histocompatibility complex (MHC) of immune competent cells. Especially, an mRNA included in the first set of synthetic mRNA molecules may encode only one or a small number of mutations whereas in the mRNA transcript detected in the patient, a larger number of mutations might be present. In another embodiment, the mRNA included in the first set of synthetic mRNA molecules may encode a peptide or protein or portion thereof which includes all mutations detected in the respective patient specific mRNA transcript. Furthermore, in the synthetic mRNA molecules an epitope can be presented flanked by amino acids which also appear in the naturally occurring peptide or protein of healthy cells, whereas in the patient, mutations are also contained in such flaking sequences. Any of such considerations and designs of the synthetic mRNA molecules can be adapted as desired and are considered encompassed by the present invention.

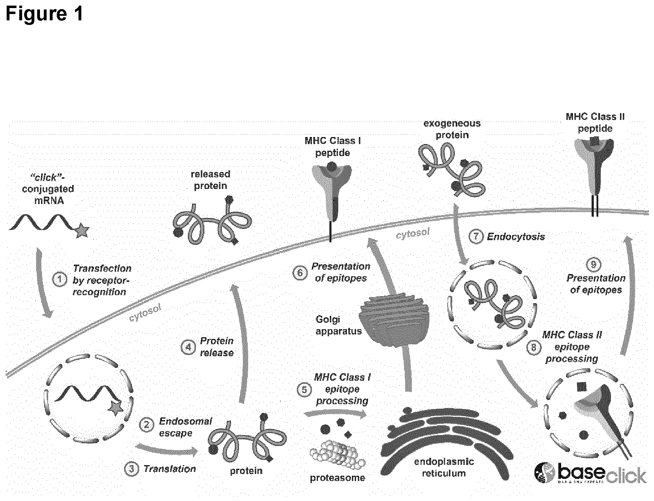

The first set of synthetic mRNA of step ii) is used to transfect immune competent cells *in vitro* in the following step iii). Tumor-specific peptides, proteins or portions thereof encoded by the first set of synthetic mRNAs are then expressed in these immune competent cells and translated into peptides or proteins. These peptides or proteins are partly released from the cells, but also partly digested by the cellular proteasome. At least part of such peptides, proteins or portions thereof and the peptides derived from digestion, i.e. appropriate epitopes, will be presented by the MHC class I or class II molecules of the immune cells. A schematic representation of the mRNA entry and the further events in immune competent cells up to presentation of the encoded peptides by MHC molecules is shown in Fig. 1.
Immune competent cells that can be used in this step iii) of the inventive method include especially all kinds of antigen-presenting cells, i.e. monocytes, macrophages, B cells and dendritic cells. While laboratory cell lines can be used, in preferred embodiments of the inventive method, immune competent cells obtained from the patient to be treated are included for this step.

It is to be understood that not necessarily all of the peptides, proteins or portions thereof encoded by the first set of synthetic mRNA molecules will have a detectable immunogenic effect. It is rather to be expected that only a part of such mRNA molecules will encode peptides or proteins which will be presented by the MHC of the immune cells and which can be candidates for vaccines. These proteins or peptides can be analyzed by appropriate methods. like for example mass spectrometry-based methods including e.g. liquid chromatography-tandem mass spectrometry or MALDI-TOF. Accordingly, in step iv), a correlation is made and tumor-specific peptides, proteins or portions thereof which are immunogenic and/or presented by the MHC, are identified, selected and included for the further steps for developing the mRNA vaccine of the invention. In this context, identification and selection can be performed according to well-known methods, e.g. enzymatic immunoassays.

These further steps include v) the production of a further set of synthetic mRNA molecules encoding the immunogenic tumor-specific peptides or proteins or portions thereof, which have been identified in step iv). According to the invention, this further set of synthetic mRNA molecules can essentially correspond to the respective selected mRNA molecules contained in the first set of synthetic mRNA molecules. However, this second set of synthetic mRNA molecules can also have different, shorter or longer sequences but still encode peptides or proteins which include the immunogenic tumor-specific peptides or proteins or portions thereof identified in step iv).

In a next step vi) of the inventive method, T cells are *in vitro* primed using said second set of mRNA molecules of step v) and an in vitro T-cell-priming assay is performed. Again, well known methods can be applied in this context, including ELISA or ELIspot assays or FACS. For step vi), naive T cells are contacted with the second set of mRNAs according to methods known in the art. In this context, the term T cell includes preferably T-helper cells (CD4+ T cells) and cytotoxic T cells (CD8+ T cells). In this context, it is again possible to use laboratory T cell lines, however, in preferred embodiments of the invention, the T cells used in step v) have been obtained from the patient to be immunized and treated. In such case, the patient's T cells might already show a certain level of immune response to the peptide or protein antigen, which however might not be strong and specific enough to kill the tumor cells or to cause degradation of the tumor. If the peptides or proteins or parts thereof, which are encoded by the second set of mRNAs trigger an immune response or intensify an existing immune response (in case that the T cells are obtained from the patient to be vaccinated), they are accordingly identified in step vii) of the inventive method.

In the final step viii) of the method of the invention, synthetic mRNA which encodes a peptide or protein identified in step vii) is produced and thus provided for use as a personalized tumor vaccine for said patient. In the context of the present invention, the mRNA vaccine can contain only one synthetic mRNA molecule encoding a peptide, protein or part thereof which triggered or increased an immune response in the T cells in step vii). In more preferred embodiments, however, several mRNAs encoding peptides, proteins or parts thereof which have been identified as triggering or increasing an immune response are included in a specific and personalized vaccine mixture. Providing different triggers in certain circumstances will result in a more pronounced immune response and a more efficient attack of the immune system against the tumor or tumor cells.

According to a preferred embodiment of the inventive method, click-modified mRNA molecules can be produced and included in at least one of steps ii), v) and viii) of the inventive method. Such click-modified mRNA molecules include at least one of an alkyne- or azide-modification in at least one nucleotide within at least one of the ORF, the 5'-UTR, the 3'-UTR and poly(A) tail region. The modification not only allows to stabilize mRNA of interest for *ex vivo* applications and also for administration to a human patient, but it also allows to easily attach detectable labels or functional groups, which - amongst other advantages - allows for targeted delivery of the modified mRNAs to specific cells or tissues and/or to monitor such delivery.

This aspect of the present invention applies so-called "click chemistry" or elements thereof and utilizes this technique to modify mRNA molecules which are provided when performing the inventive method. mRNA stability and translation efficiency depend on several factors. Especially the untranslated regions at either ends of the mRNA play a crucial role. In eukaryotic protein expression, a cap structure at the 5'-end and the poly(A) tail at the 3'-end both increase mRNA stability and enhance protein expression. In addition, the 5'-UTR contains a ribosome binding site necessary for translation and the 3'-UTR contains RNA sequences that adopt secondary structures which improve stability and influence translation. Moreover, modified natural, e.g. *N*1-methylpseudouridine, and artificial nucleotides can be incorporated to improve mRNA stability and enhance translation of the mRNA (Svitkin Y.V. et al., Nucleic Acids Research, Vol. 45, No. 10, 6023-6036 (2017)).

Delivery of mRNAs into cells can be achieved by providing mixtures containing lipids for fusion with the cellular membrane and cations to neutralize the negative charge of the oligonucleotide backbone. Special formulations have been created to optimize mRNA delivery and to confer sufficient *in vivo* stability for clinical trials. Most of the mRNA formulations which are applied intravenously are taken up by and are expressed inside liver cells. This is due to the fact that the liver plays a major role in fatty acid metabolism and a high lipid content of the mRNA formulations therefore displays an organ-specific targeting effect. In most cases the liver is, however, not the desired target and therefore efforts are being made to modify lipid formulations to target organs, which are involved in an immune response, like e.g. the spleen (Kranz L.M. et al., Nature 534, 396-401 (2016)). Alternatively, cells of the immune system (e.g. lymphocytes) can be isolated from a patients' blood and mRNA application is performed *ex vivo* to allow targeting.

Click chemistry is a concept which was defined in 2001/2002 by the groups of Sharpless and Meldal (Sharpless, K.B. et al., Angew. Chem. 2002, 114, 2708; Angew. Chem. Int. Ed. 2002, 41, 2596; Meldal, M. et al., J. Org. Chem. 2002, 67, 3057). Since then, especially the copper catalyzed reaction of azides with alkynes to give 1,2,3-triazoles, a variation of the 1,3-dipolar Huisgen cycloaddition (R. Huisgen, 1,3-Dipolar Cycloaddition Chemistry (Ed.: A. Padwa), Wiley, New York, 1984), has become a very widely used method to perform a click reaction. As a result of its mild conditions and high efficiency, this reaction has found a myriad of applications in biology and material sciences, such as e. g. DNA labeling for various purposes (Gramlich, P.M.A. et al., Angew. Chem. Int. Ed. 2008, 47, 8350).

In this context, it is specifically referred to WO 2019/063803 A1, the disclosure of which is included for the purposes of the present invention, especially as far as it relates to modified mRNA, methods for producing such modified mRNA as well as conditions, reagents and methods to perform click reactions. In addition to the copper-catalyzed click-reaction, also copper-free, bio-orthogonal methods have been developed and such methods can also be employed in the context of the present invention. E.g., strain-promoted azide-alkyne cycloaddition (SPAAC) (I.S. Marks et al., Bioconjug Chem. 2011 22(7): 1259-1263) or inverse electron-demand Diels-Al-der cycloaddition (iEDDA) (D. Ganz et al., RSC Chem. Biol. 2020 1 (3): 86-97) can be used either alone or in combination with copper-catalyzed click chemistry (Cu-AAC) in the context of the present invention. Especially in cases in which it is desirable to perform a labelling reaction *in vivo* in cell culture or in a living organism, performing such reaction using SPAAC or iEDDA is preferable since these methods do not require the use of toxic substances or external catalysts. Accordingly, as far as in the following or via the above reference to WO 2019/063803 alkyne- and azide-modifications are considered or mentioned, within the scope of the present invention these terms are to be understood to include reagents employed in the context of such bio-orthogonal click-reactions, especially alkenes, preferably trans-cyclooctenes, and tetrazines.

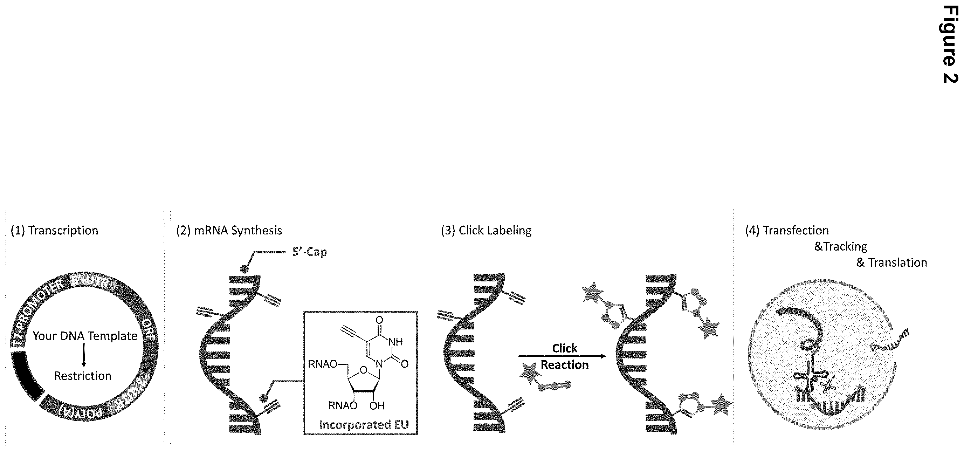

Click chemistry facilitates attaching reporter molecules or labels to biomolecules of interest and is a very powerful tool for identifying, locating, and characterizing such biomolecules. The method for example enables inclusion and attachment of fluorescent probes for spectrometric quantification, or of anchor molecules to allow for separation and purification of the target biomolecules. Up to date, many applications have been developed in which click chemistry is used as an underlying principle. Next-generation sequencing is one of such applications which benefits from this technique where formation of so-called "backbone mimics", i.e. non-natural alternatives for the phosphodiester bond, which can be generated by copper-catalyzed azide alkyne cycloaddition (CuACC), is used to ligate e.g. DNA fragments and adapter sequences. Despite the presence of a triazole ring instead of a phosphodiester bond, such backbone mimics are acceptable substrates for polymerase driven DNA or RNA preparation methods like PCR or reverse transcription. A schematic representation of synthesis, labeling, cell transfection, tracking and protein expression of modified mRNA is shown in Fig. 2

The alkyne- or azide-modification can be included in only some or all elements contained in the mRNA, and needs to be included in at least one of the UTRs, ORF and poly(A) tail. The 5'-cap structure preferably does not contain such alkyne- or azide modifications, as changes to the cap structure can interfere with efficient binding of initiation factors like elF4E, elF4F and elF4G and thus drastically decrease translation efficiency.

The presence of such modification on the one hand stabilizes the mRNA and on the other hand provides specific anchor sites for labeling or for attachment of tissue- or cell-specific ligands or targeting molecules via click chemistry. Thus, using click-modified mRNA in the context of the present invention not only enables detection of the presence and the location of mRNA after transfection and/or priming of cells in steps iii) and vi), but also provides new options for targeted delivery of mRNAs to specific cells in steps iii), vi) and also within the context of the vaccination of a patient in step viii).

Depending on which type of nucleotide or nucleotides are included in alkyne- or azide-modified form during mRNA production, the resulting modified mRNA can contain modifications not only in the 5'- and 3'-UTRs and the ORF, but also in the poly(A) tail region. As apparent to the skilled person, including e.g. one or more of modified CTP, GTP and UTP leads to a modification within the UTRs and the ORF, while additionally including modified ATP results in a modification also of the poly(A) tail region. Including only alkyne- and / or azide-modified ATP during the transcription leads to modifications in the UTRs, the ORF and the poly(A) tail region.

No negative effects caused by the presence of alkyne- or azide-modified nucleotides within the mRNA have been observed. The modification of the mRNA does not seem to impair translation of mRNA during protein production at the ribosomes. Depending on the circumstances, the amounts of modified nucleotides to be included in the mRNA can be adjusted. For instance, when a dye is to be attached to the mRNA as a detectable label via a click reaction, it might be desirable to include an adequately high amount thereof to ensure and facilitate detection, whereas in order to target specific cell receptors, it might be sufficient to include only one or a few respective ligand molecules to achieve the desired effect.

As mentioned, the inclusion of alkyne- or azide-modified nucleotides has a stabilizing effect on mRNA. It is to be expected that the stabilizing effect of the modification is most pronounced if such modification is distributed over the complete mRNA molecule. In such case, subsequent attachment of detectable labels and/or functional molecules via click chemistry will occur also uniformly over the whole mRNA molecule and can even provide for an enhanced stabilizing effect.

However, in some cases it may be important to restrict the inclusion of labels or functional molecules to a part of the mRNA molecule which is not involved in subsequent translation of mRNA during protein expression. For such purpose, it can be desirable to include modified nucleotides in the poly(A) tail region only whereby it can be ensured that ribosomal activity is not impaired by the presence of especially longer or bulkier labels or functional molecules like ligands or targeting molecules.

The present invention therefore also includes using a modified mRNA containing alkyne- or azide-modification in the poly(A) tail region only. Instead of including alkyne- or azide modified nucleotides during e.g. *in vitro* transcription of a DNA template or in a fermentation process, a modification in only the poly(A) tail region can easily be achieved for any desired mRNA by performing an addition reaction in the presence of poly(A) polymerase and alkyne- or azide-modified ATP.

By controlling the amount and type of alkyne- or azide-modification in the modified mRNA, it is possible to conveniently and easily adapt the resulting mRNA to impart stabilization and options for attachment of molecules of interest as required and viable with regard to the respective steps of the method of the present invention.

The azide- or alkyne-modification can be included at the nucleobase or at the 2'-position of the ribose unit of the respective nucleotide. In very special cases, inclusion of a nucleotide containing the modification at the 3'-position of the ribose is also possible. In such case, the enzymatic poly(A) addition reaction is terminated upon inclusion of one modified nucleotide. In one embodiment, the modified mRNA can contain an alkyne- and/or an azide-modification at the nucleobase or the 2'-ribose position in at least one of nucleotides within at least one of the UTRs, the ORF and optionally also the poly(A) tail region, and additionally a chain-terminating alkyne- or azide-modification at the 3'-position of the ribose in the poly(A) tail. In a different preferred embodiment, the mRNA does not contain a chain-terminating alkyne- or azide-modification at the 3'-ribose position in the poly(A) tail region.

The modified nucleotide included in the mRNA can be derived from a natural nucleotide and especially one of the standard nucleotides with adenine, cytosine, guanine or uracil bases, or it can be a modification of another naturally occurring nucleotide (e.g. pseudouridine derivative) or even a non-naturally occurring molecule (e.g. F. Eggert, S. Kath-Schorr, Chem. Commun., 2016, 52, 7284-7287) which does not negatively affect transcription and/or translation and the function of the resulting modified mRNA. Preferably the modified nucleotide is derived from a natural nucleotide or a naturally occurring nucleotide within mRNA.

Suitable alkyne- and azide-groups for click reactions are known and available to the skilled person and all such groups can be used to prepare modified mRNAs to be used in the context of the present invention. The alkyne-modified nucleotide preferably is an ethynyl-modified nucleotide, more preferably 5-ethynyluridine phosphate or 7-ethynyl-7-deazaadenine phosphate, or a cyclooctynyl-modified nucleotide (for an SPAAC type click-reaction). While it is also possible to employ higher straight-chained alkyne-modified nucleotides, especially propynyl or butynyl modified nucleotides and C-C triple bond-containing ring systems, possible negative effects on the translation of the mRNA into a peptide or protein will have to be considered when selecting suitable alkyne molecules.

Azido-modifications for nucleotides which are useful in the context of the present invention can, e.g., also include azidoalkyl groups in which the alkyl part preferably is a lower alkyl group, especially a methyl, ethyl or propyl group. As an azide-modified nucleotide, preferably 5-(3-azidopropyl)-uridine phosphate or 8-azidoadenine phosphate are considered for inclusion in the inventive mRNA. An example for an azide-modified nucleotide causing termination of the poly(A) addition reaction is 3'-azido-2',3'-dideoxy adenine phosphate.

In the context of the present invention, in a modified mRNA molecule at least one of the four standard types of nucleotides (AMP, CMP, GMP and UMP) is partly or completely modified, preferably ethynyl, cyclooctynyl- or azido-modified at uracil or adenine. In a further preferred embodiment, a modified mRNA molecule contains at least one alkyne- modification and at least one azide-modification.

In principle, all nucleotides of the at least one type of modified nucleotide can be alkyne- or azide-modified, or alternatively, only a part of such nucleotides is present in modified form. In a preferred embodiment of the present invention and depending on the desired modification and modification rate, ratios of modified versus non-modified forms of the various nucleotides can range from 1:100 to 10:1, preferably 1:10 to 10:1, further preferably 1:4 to 4:1, and also preferably 1:2 to 2:1. Preferably, a 1:1, 1:4 or 1:10 combination of modified to non-modified nucleotide is included in the mRNA.

As mentioned above, the presence of alkyne-or azide-modified nucleotides or nucleobases in the modified mRNAs of the present invention confers a stabilizing effect. On the one hand, the attack of endoribonucleases is restricted to some extent by an internal modification. Extension of the poly(A) tail region during poly(A) polymerase based addition of modified ATPs at the 3'-end leads to a further stabilizing effect. Attack on and degradation of mRNA molecules by exoribonucleases occurs at the two ends of RNA. The mRNA molecules used according to the invention contain a cap at the 5'-end which provides protection from degradation at that side. Including additional modified adenosine nucleotides at the 3'-end imparts further protection as the attack of exoribonucleases in 3'→5' direction is impeded and degradation reaching the core mRNA, especially the ORF, is delayed.

In another preferred embodiment of the present invention, detectable labels and / or functional molecules can be introduced into the modified mRNA via click reaction with a correspondingly modified alkyne- or azide-containing label or functional molecule. As for the nucleotide modification, also for the modification of detectable labels or functional groups, suitable alkyne- and azide groups are known to the skilled persons and the preferred examples for such groups are applicable as described above. The reaction of an alkyne-modified nucleotide within the modified mRNA and an azide-containing label or functional molecule or the reaction of an azide-modified nucleotide within the modified mRNA and an alkyne-containing label or functional molecule is performed under conditions to conduct the click reaction and leads to formation of the 5-membered heterocyclic 1,2,3-triazole moiety which forms the link between mRNA and label or functional molecule. According to the present invention, the term alkyne-containing label or functional molecule also encompasses C-C triple bond-containing ring systems like cyclooctynes which have been considered especially in the context of SPAAC reactions and *in vivo* labelling via bio-orthogonal ligation reactions.

The type and size of labels and functional molecules are not particularly restricted as long as they do not negatively affect the translation efficiency of the mRNA and, especially in case of delivery of vaccine mRNA, integration of the mRNA into immune cells. Preferred examples for the detectable label include color imparting or a fluorescence imparting labels, e.g. fluorescein derivatives like FITC, Alexa Fluor dyes or DyLight Fluor dyes, cyanine dyes like Cy5 and Cy3 or rhodamine dyes like Texas Red and 5-TAMRA, or any other fluorescent dye. Inclusion of such labels can be especially valuable to determine the efficiency of the *in vitro* transfection and priming steps in steps iii) and vi). Including such labelled mRNA molecules can be useful for evaluating the immunogenic activity of the mRNA encoded proteins or peptides by assessing the transfection efficiency.

Functional molecules to be included in the modified mRNA *via* a click reaction are not restricted and are preferably cell- or tissue-specific ligands that mediate targeted uptake of the mRNA into the immune cells and especially the T cells during steps iii) and vi). Furthermore, delivery of the mRNA vaccine to cancer cells or at least allow to attach or anchor the mRNA onto the cell-surface is a further advantage of the use of modified mRNAs in step viii).

Such cell- or tissue-specific targeting can be achieved for example by using certain (small) peptides, sugar moieties, small molecules (e.g. folic acid) or fatty acid moieties as the cell- or tissue-specific ligands. Respective substances have been described for a large number of targeting applications and are available to the skilled person (Hu, B., Therapeutic sIRNA: state of the art., Signal Transduction and Targeted Therapy, 2020:5:101). Some preferred and exemplary targeting molecules are receptor ligands which target cell specific receptors like e.g. the epidermal growth factor receptor, folate which targets the folate receptor, apolipoproteins which target endogenous low-density lipoprotein receptors, arachidonic acid which targets the endogenous cannabinoid receptors or GalNac which targets receptors e.g. on hepatocytes (Willoughby, J. L. S.; et al. Evaluation of GaINAc-SiRNA Conjugate Activity in Pre-Clinical Animal Models with Reduced Asialoglycoprotein Receptor Expression. 2018, 26, 105-114.). Also, the amino acid sequence RGD or similar sequences have been found to mediate cell adhesion and can also be considered as preferred ligands within the context of the present invention. Furthermore, combination of different targeting molecules and/or small molecules and natural oligomers can be employed.

The presence of functional molecules attached to the mRNA can further increase mRNA stability against nuclease degradation and it has been shown that partial as well as full replacement of at least one of the natural nucleotides within the mRNA for an alkyne- or azide-modified analogue and even attachment of functional molecules thereto usually does not hamper translation of the mRNA molecule.

In addition to including either alkyne-modified or azide-modified nucleotides, it is also possible that a modified mRNA used in the context of the inventive method contains at least one nucleotide in partially or completely alkyne-modified form and at least one other nucleotide in partially or completely azide-modified form. A further option is the use of an mRNA including at least one type of nucleotide in partially or completely alkyne- as well as in partially or completely azide-modified form. Such mRNA contains two different anchor modifications to which different functional molecules can be attached in a downstream post-enzymatic click reaction. For example, but without limiting to such specific embodiment, a first alkyne-modified cell-specific targeting group as well as a second different azide-modified targeting group can be attached resulting in another preferred embodiment of an mRNA which can be used in the context of the present invention which provides for maximum specificity and/or effectivity of cell targeting and mRNA uptake into the cells.

In summary, the use of modified mRNAs as described above leads to significant advantages within the overall method of the present invention. It is not only the stabilization of mRNA and accordingly a longer half-life and presence of the mRNA in the *in vitro* and *in vivo* applications, but also the enhanced targeting and transfection efficiency which can be achieved in the context of this preferred embodiment of the present invention.

As explained above, step i) of the inventive method requires that a genetic analysis is performed in order to identify the specific mRNA transcripts which are produced in a tumor cell. This first step in the inventive method also applies to identifying tumor-specific peptides or proteins which are encoded by said tumor-specific mRNA transcript. In certain embodiments of the present invention, such analysis might have been performed already before a specific mRNA-tumor vaccine is produced. In such embodiments which are included within the scope of the present invention, step i) does not need to be performed directly and in connection with steps ii) to viii) of the inventive method. Under such circumstances, step i) is considered optional or as having been performed as a preparatory step well in advance of other steps of the inventive method.

In preferred embodiments of the present invention, however, the inventive method includes step i) and the genetic analysis comprises sequencing of mRNA contained in a tumor-specific sample obtained from the patient and performing a transcriptome analysis to detect tumor-specific mRNA molecules. In especially preferred embodiments of the present invention, the genetic analysis comprises the preparation of a full-length mRNA library of the sample obtained from the patient.

Despite all technical advancements in recent years, RNA sequencing, especially full-length mRNA sequencing or transcriptome or exome sequencing has remained a tremendous challenge. A major problem in this context is the fact that very often only small biological samples are available for the sequencing. Such samples provide the various components of the genetic material only in minute amounts. In order to reach the required sensitivity, an amplification step is usually required in which the amount of the nucleic acids in the sample is increased prior to analysis. The amplification of full-length RNA from natural sources, especially of full-length mRNA, the total mRNA of specific cells or cell types, or even the whole exome of an organism, i.e. the complete coding regions of protein encoding genes, via commonly used methods is still a complicated and time-consuming procedure. Within the context of the present invention, an improved concept for amplification of mRNAs and for preparation of full-length libraries especially of the plurality of mRNAs contained in a sample can be used as described below. This improved concept allows the whole exome preparation of the total mRNA of the cell sample of an individual with limited effort and improved results.

According to this preferred embodiment of the invention the genetic analysis performed in step i) of the inventive method comprises
a) providing at least one first primer, which first primer includes a sequence which is complementary to a sequence which is located at or near the 3'-end of the at least one RNA to be amplified, and adding said at least one first primer to the sample under conditions which allow for hybridization of at least one first primer to at least one RNA,
b) reverse transcribing the at least one RNA under conditions including addition of a reverse transcriptase and nucleotides to the sample to provide full length cDNA(s) of the at least one RNA,
c) purifying the sample at least from excess nucleotides
d) adding a dideoxy nucleotide, which dideoxy nucleotide is modified at the 3'-position to include a first partner of a pair of azide and alkyne molecules, and a template-independent polymerase to attach a single 3'-azide- or 3'-alkyne modified dideoxy nucleotide at the 3'-end of the cDNA(s) obtained in step b),
e) purifying the sample at least from excess modified dideoxy nucleotide added in step d),
f) adding an adapter molecule, the adapter molecule comprising a polynucleotide sequence and, attached to its 5' end, a second partner of said pair of azide and alkyne molecules, under conditions to perform a click reaction and to ligate the adapter molecule to the 3'-modified cDNA(s) obtained in step d) under formation of a triazole linkage,
g) adding a second primer, the second primer comprising a nucleotide sequence which is complementary to at least 6 of the nucleotides at the 5'-end of the adapter molecule and contains at its 3'-end a nucleotide which is complementary to the dideoxy nucleotide at the 3'-end of the cDNA(s), to effect hybridization and binding of the second primer to the ligated adapter/cDNA molecule obtained in step e) at a position overlapping the triazole linkage,
   and alternatively
h) adding a DNA polymerase to achieve chain extension of the second primer to produce second strand DNA(s) including sequences complementary to the whole cDNA(s) obtained in step b), and either
i) sequencing the second strand DNA(s) of step h), or
j) adding a third primer which is identical to at least a part of the first primer, and amplifying the full-length cDNA(s) to obtain a full length RNA library,
   or
h') adding a DNA polymerase to achieve chain extension of the second primer and simultaneously determining the sequence of the second strand DNA(s) and the whole cDNA(s).

In a second alternative of this preferred embodiment, the genetic analysis performed in step k) of the present invention comprises:
a) providing at least one first primer, which first primer includes a sequence which is complementary to a sequence which is located at or near the 3'-end of the at least one mRNA to be amplified, and which primer contains a modification by a first partner of a pair of azide and alkyne molecules at its 5' end, and adding said at least one first primer to the sample under conditions which allow for hybridization of at least one first primer to at least one mRNA,
b) reverse transcribing the at least one mRNA under conditions including addition of a reverse transcriptase and nucleotides to the sample to provide full length cDNA(s) of the at least one mRNA,
c) purifying the sample at least from excess nucleotides
d) adding a dideoxy nucleotide, which dideoxy nucleotide is modified at the 3'-position to include a second partner of a pair of azide and alkyne molecules, and a template-independent polymerase to attach a single 3'-azide- or 3'-alkyne modified dideoxy nucleotide at the 3'-end of the cDNA(s) obtained in step b),
e) purifying the sample at least from excess modified dideoxy nucleotide added in step d),
f) performing a click ligation reaction to generate circular single stranded cDNA(s) under formation of a triazole linkage,
g) adding a second primer, the second primer comprising a nucleotide sequence which is complementary to at least 6 of the nucleotides at the 5'-end of first primer and contains at its 3'-end a nucleotide which is complementary to the dideoxy nucleotide at the 3'-end of the cDNAs, to effect hybridization and binding of the second primer to the ligated circular first primer/cDNA molecule(s) obtained in step f) at a position overlapping the triazole linkage,
   and either
h) adding a DNA polymerase to achieve chain extension of the second primer and subsequently amplify the full-length cDNA(s),
   or
h') adding a DNA polymerase to achieve chain extension of the second primer and simultaneously determining the sequence of the circular single stranded DNA(s) including the whole cDNA(s).

The method described herein employs "click chemistry" to facilitate amplification of a full-length RNA, and even the amplification of several or a multitude of RNAs contained in a sample can be performed in one single procedure. Determination of the sequence of tumor specific mRNAs within the context of the present invention can be performed either after amplification or even during second strand synthesis of cDNA(s) obtained by reverse transcription of the RNA(s).

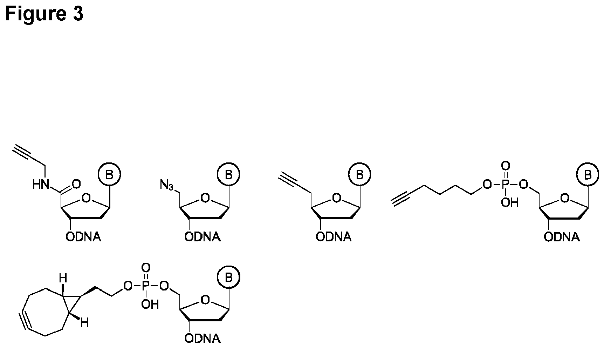

In principle, the method can be applied to all kinds of RNA, if amplification and/or sequencing of such RNA and especially of a mixture containing any kind of RNA is desired. Within the context of the present invention, however, mRNA is the target of this preferred amplification and/or sequencing process. Examples of click-ready 5'-alkyne- or azide-modified oligonucleotides for use in the context of this method are outlined in Fig. 3. The length of the spacer between the alkyne triple bond or the azide motif and the thus modified nucleotide can vary and is only limited by the ability of a DNA polymerase or a reverse transcriptase to read through the respective template comprising a triazole linkage resulting from the click reaction of a pair of alkyne and azide molecules. In preferred embodiments of the invention, the spacer comprises at least one atom and at most 30 atoms, more preferably at most 20 atoms and most preferably at most 10 atoms. The spacer includes C atoms, however can also comprise heteroatoms N and/or O.

Shortly summarized, the first step within this amplification/sequence determination includes cDNA preparation from the mRNA obtained from the patient's sample via reverse transcription, during or after which the obtained cDNAs are modified at their 3'-end to include an alkyne- or an azide-modification. More specifically, one partner of a pair of molecules which participate in a click reaction is attached to the cDNA(s). Subsequently, an adapter comprising a poly- or oligonucleotide sequence and, at its 3'-end, the second partner of the pair of molecules, is ligated via click reaction to the cDNA(s). Use of this adapter and a specific second primer allow for a highly effective amplification of the cDNAs or for a direct sequencing of the cDNAs.

This first alternative for the amplification/ sequencing of mRNAs includes an initial step a), in which a first primer which is complementary to a sequence which is located at or near the 3'-end of the at least one mRNA, is added to the sample containing RNAs. Addition of this first primer to the sample occurs under conditions which allow for hybridization of the first primer to the complementary mRNA sequence.

The at least one first primer includes a sufficient number of nucleic acids which are complementary to the respective target mRNA to allow for an effective hybridization of the primer to the mRNA. The first primer can include any number of nucleotides which is considered useful in DNA/RNA amplification procedures. In a preferred embodiment, the first primer consists of at least 5 nucleotides, preferably at least 10 or at least 15 nucleotides. In such preferred embodiments, the first primer further consists of up to 60 nucleotides, preferably up to 50 nucleotides and most preferably of up to 45 nucleotides. Conditions to effect or favor hybridization of the at least one first primer to the at least one mRNA are well known to the skilled person and include those as described e.g. in J. Zhang et al., Biochem. J.,1999, 337, 231-241. Especially favorable conditions can also easily be determined by the skilled person by preliminary tests.

In a second step b) of this first inventive method, starting from the 3'-end of the primer hybridized to the at least one mRNA, the mRNA is reverse-transcribed under conditions including addition of a reverse transcriptase and nucleotides to the sample to provide full-length cDNA(s) of the at least one mRNA. While the naturally occurring 4 types of nucleotides dATP, dCTP, dGTP and dTTP are usually employed in the context of the present invention, also artificial nucleotides can be included as long as they do not impair the cDNA formation of any further steps of the method of this invention. As for step a), conditions for the reverse transcription are also well known to the skilled persons.

In an intermediate step c), at least excess nucleotides used in step b) are removed. In preferred embodiments of the invention, also remaining mRNA(s), excess reverse transcriptase and excess first primer(s) can be removed during this step. In an especially preferred embodiment, a cDNA purification is performed according to methods known in the art and the purified cDNA included in the further steps of the method.

In the following step d), a dideoxy nucleotide (ddNTP), which is modified at the 3'-position to include a first partner of an azide/alkyne click-reaction pair, and a template-independent polymerase is added to the sample. The term "dideoxy nucleotide" or ddNTP stands for nucleotides which do not have 2'- nor 3'-hydroxyl groups.

Within the context of the present invention, the ddNTPs include one of the bases adenine, thymine, cytosine or guanine. The modified dideoxy nucleotide is added to the cDNA sequence at its 3'-end by the polymerase and, since the dideoxy nucleotide does not contain the required 3'-hydroxyl group, no further chain extension can occur and the attached azide or alkyne group is available for a reaction of the 3'-end of the cDNA with a second click-reaction partner. In a preferred embodiment of the invention, the alkyne or azide group is attached to the 3'-position of the deoxy-ribose moiety of the ddNTP and also preferably, the ddNTP contains an azide group attached to the molecule.

In a further intermediate step e), at least an excess of the modified dideoxy nucleotide used in step d) is removed. In preferred embodiments of the invention, also during this step eventually remaining mRNA, excess reverse transcriptase and excess first primer(s) can be removed and/or the template-independent polymerase as used in step d).

In step f), an adapter molecule is added as the second click-reaction partner to the sample. The adapter molecule comprises an oligo- or polynucleotide sequence and contains attached to its 5'-end the second partner of the pair of azide and alkyne molecules. A click-reaction which occurs between the alkyne- and the azide groups ligates the adapter molecule to the 3'-end of the cDNA under formation of a triazole linkage. The adapter molecule serves for binding of a second primer in the next step of the inventive method via hybridization and subsequent amplification. Accordingly, the adapter molecule contains a number of nucleotides which is sufficient to allow for binding of such second primer, usually at least 6 nucleotides, preferably at least 10 and more preferably at least 15 nucleotides. In a preferred embodiment, the adapter molecule contains a maximum of 100 nucleotides, more preferable a maximum of 80 or 60 nucleotides. Furthermore, it is also considered a preferred embodiment of the invention to use an adapter molecule in step e, which contains an alkyne attached to its 5'-end.

In the next step g), the above mentioned second primer is added which comprises a nucleotide sequence, which is complementary to all or part of the adapter molecule. The nucleotide sequence of the second primer is complementary to at least 6 nucleotides at the 5'-end of the nucleotide sequence of the adapter molecule and it contains at its 3'-end a nucleotide which is complementary to the dideoxy nucleotide at the 3'-end of the cDNAs. Accordingly, the second primer hybridizes and binds to the ligated adapter/cDNA molecule at a position which overlaps the triazole linkage which was formed via click reaction.

In the further steps, the second primer is extended by addition of a DNA polymerase. As far as an amplification of the one or more RNAs is desired, one or more third primers can be added which are identical to at least a part of the first primer(s), and the obtained cDNAs then can be amplified according to known methods. This option is defined by steps h) and j). The third primer can e.g. comprise less nucleotides than the first primer but still ensure hybridization. The third primer can also include a specific sequence which ensures hybridization and further sequences which are not complementary to the target sequence of the first primer on the at least one mRNA.

As far as sequencing of the mRNAs is desired, there are different options within the context of this method. Firstly, after step g), a DNA polymerase can be added to achieve chain extension of the second primer to produce "full length" second strand DNAs including sequences which are complementary to the whole cDNAs produced in step b). These second strand DNAs can be sequenced without being amplified according to known methods for single molecule sequencing (system and kits available e.g. from Pacific Biosciences, https://www.pacb.com/). This option is defined in steps h) and i).

In an alternative embodiment, the sequencing can be performed simultaneously with chain extension of the second primer after adding a DNA polymerase. This alternative option is defined by step h'). The sequencing can e.g. be performed according to the well known Sanger sequencing method or methods developed based on this principle, or a sequencing-by-synthesis method (systems and kits e.g. available from Illumina, https://illumina.com/).

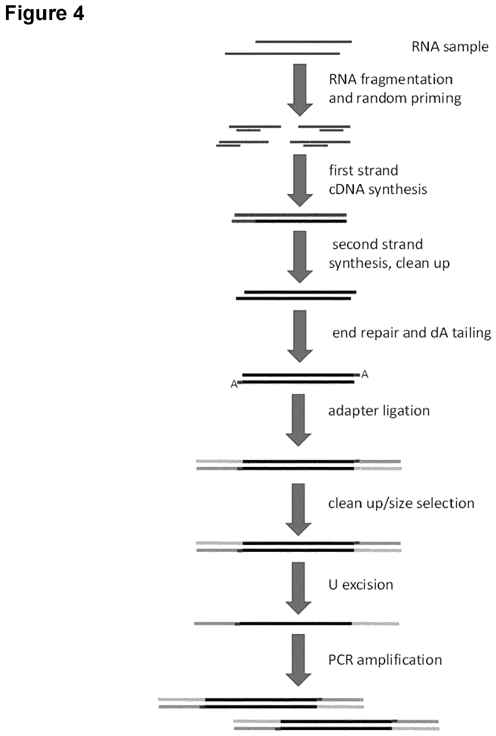

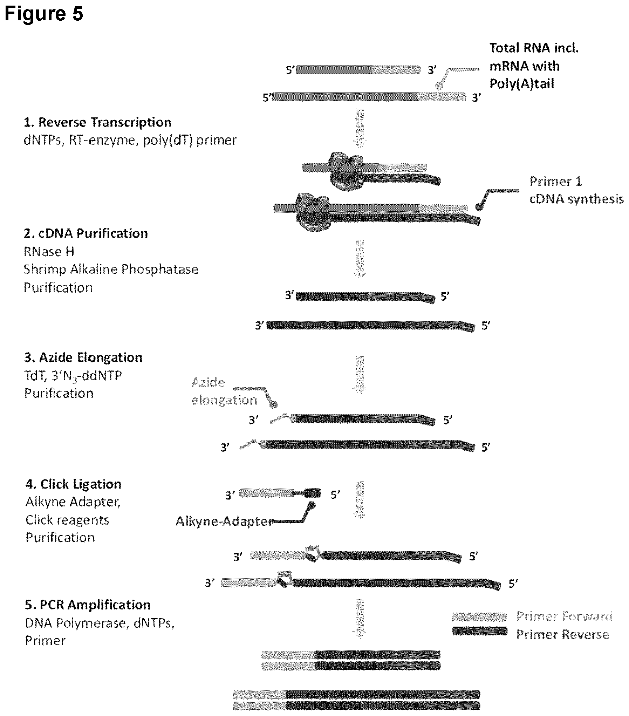

The above described method allows to produce amplified cDNA for a specific RNA sample without the need for fragmentation of the RNA and random priming as was required by commonly used processes, one of which is schematically represented in Fig. 4. Compared to such prior art amplification processes, the method as herein disclosed requires less process steps to arrive at a fully representative cDNA library for e.g. the complete mRNA or mRNAs of interest without the need for aligning sequence data for overlapping fragments. Also, the method produces full-length libraries from either random priming or specific priming. Additionally, the method can be carried out with standard polymerases for library preparation workflows. A schematic representation of a method of the invention using poly(dT) priming is shown in Figs. 5.

As the first primers, various different alternatives and respective polynucleotides can be considered. Firstly, random primers can be used as known from prior art methods. For this first alternative, rather short nucleotide sequences having a random sequence which hybridizes to random parts within the mRNAs can be employed. The products of a process using such random first primers have different lengths.

In a further alternative which is also a preferred embodiment, a poly(dT) primer is used as the at least one first primer for mRNA amplification. A poly(dT) primer usually comprises 6 to 30 dT nucleotides. The primer is added to the sample under conditions which allow for hybridization to the poly(A) tail(s) of the at least one mRNA in the sample. All mRNA molecules contain a poly(A) tail at their 3'-end which consists of multiple adenosine monophosphates. The poly(A) tail is added to eukaryotic RNA via polyadenylation which takes place after the transcription of the gene is concluded. The poly(A) tail however does not have a coding function during translation. Thus, the poly(A) tail is an ideal target for primer hybridization and providing a corresponding poly(dT) primer does not require any knowledge about the sequence of the target mRNA. Furthermore, use of the poly(dT) primer allows for reverse transcription and amplification of the complete coding sequence of the mRNA(s) contained in a sample.

In certain embodiments, the method includes the use of different kinds of primers, i.e. random and specific primers or random and poly(dT) primer or specific and poly(dT) primers. This is especially useful in cases where primers also include a specific anchor or capture sequence. For instance, a specific primer can be included for detecting the presence of a known mRNA in a sample. The presence e.g. of a capture sequence attached to the 5'-end of such specific primer provides the option to, in a later step after amplification, attach the amplified cDNA obtained for this known mRNA to a solid support, to separate the cDNA attached to the solid support from other amplification products and to detect the presence of such cDNA. Other useful options provided by the inclusion of capture or anchor sequences attached to the first primer(s) are easily recognizable by the skilled person and are also encompassed within the context of the present invention.

In an especially preferred embodiment of the method for mRNA amplification, a poly(dT) primer is used as the at least one first primer, which includes an anchor sequence. This anchor sequence provides an additional 5'-end-extension of usually 2 to 50 nucleotides. Preferably the anchor sequence contains at least 5 and more preferably at least 8 nucleotides. In further preferred embodiments, the anchor sequence contains a maximum of 40, more preferably a maximum of 30 nucleotides. This presence of such anchor sequence e.g. provides a possibility to ensure uniform positioning of the poly(dT) primer(s) used in this method.

In a further preferred embodiment, 3'-alkyne- or 3'-azide-modified ddGTP or 3'-alkyne- or 3'-azide-modified ddCTP is added in step d) of the method. It has been observed that the presence of one of the bases G or C at the 3'-end of the cDNA results in an especially efficient hybridization and attachment of the second primer in the following step g). The best binding results were obtained where the 3'-end of the cDNA is modified to include G by adding a 3'-alkyne- or 3'-azide modified ddGTP to the cDNA in step d), the most preferred modified dideoxy nucleotide being 3'-azido-2',3'-dideoxy GTP (AzddGTP).

In correspondence with the above mentioned preferred embodiments for step d), it is further preferred that the second primer in step g) contains at its 3'-end dG or dC, most preferably dC. The method of the present invention requires the second primer to contain at its 3'-end a nucleotide which is complementary to the nucleotide at the 3'-end of the cDNA(s). Accordingly, in the preferred embodiment, in which an alkyne- or azide-modified ddGTP is used in step d), the second primer contains dC at its 3'-end. In the other preferred embodiment, in which alkyne- or azide-modified ddCTP is included in step d), the second primer correspondingly contains dG at 3'-end. In line with the above disclosed most preferred embodiment using the modified ddGTP above, in such most preferred embodiment a second primer is used which contains dC at its 3'-end. In a further preferred embodiment of the present invention, the second primer includes a dC at the second position of its 3' end. It has been realized that the presence of dC at this position, where the second primer overlaps the triazole linkage formed by the click reaction, improves primer binding and subsequent primer extension considerably.

As the template independent polymerase in step d) preferably the enzyme terminal deoxynucleotidyl transferase (TdT) is used. The enzyme is a member of the X family of DNA polymerases and catalyzes the addition of nucleotides to the 3'-terminus of DNA molecules. Unlike most other DNA polymerases, TdT does not require a template. It can add nucleotides to a 3'-overhang as a preferred substrate, however, also works well in adding nucleotides to blunt or recessed 3'-ends. Accordingly, it has found important and wide spread application in molecular biology, i.e. in rapid amplification of cDNA ends (RACE), PCR and more recent enzymatic *de novo* gene synthesis.

In the context of the present invention, it is an option and also a preferred embodiment to include at least one of the primers and the adaptor molecules which include a moiety which comprises a detectable label or tag, or which allows for immobilization on a solid support. One of such options has already been described above for the first primer(s). Such moiety can for example be able to form a covalent bond to a solid support directly or indirectly via further molecules. For example in cases in which it is intended to capture amplified nucleic acids for further detection or sequencing, immobilization to a solid support can be highly advantageous and is commonly used in automated sequencing methods. In line with such embodiment, one of the primers or the adaptor used in the present invention can also be present already attached to a solid support and the method be performed in the presence of such solid support. As a solid support, any material can be included or added directly or at a later stage which enables capturing and also separation of the attached molecules from the reaction mixture. As solid support, a variety of materials and molecules can be included and used within the context of the present invention. E.g., flat or three-dimensional surfaces can be provided as a solid support, e.g., beads to which a primer or adaptor is attached or can be attached via subsequent binding reaction, can be used advantageously in this context. Furthermore, it is also possible to use one of the primers or the adaptor in a labelled form, i.e. including a detectable label or tag to allow for the detection of amplification products. Especially in cases, where specific primers are used, a corresponding detection of the presence of the amplified cDNAs is easy to be carried out by methods known to the skilled person.

Also, the above outlined options can be combined to provide for capture and detection of the amplified cDNAs. In an especially preferred further embodiment, at least one of the primers or adaptor is attached to a solid support or is connected or bound to a molecule which facilitates binding to a solid support, and at least one other of the primers and adaptor molecules contains a detectable label or a molecule via which a detectable label can be attached. These embodiments allow for a simple and preferably also automated detection of amplification products in the sample to be analyzed.

While the above described preferred examples cannot exhaustively list all possible variations and benefits of the method, the skilled person can easily adapt the method to best suit the specific purpose of research or diagnosis. Especially combinations of the method with further known procedures are therefore included within the scope of this disclosure.

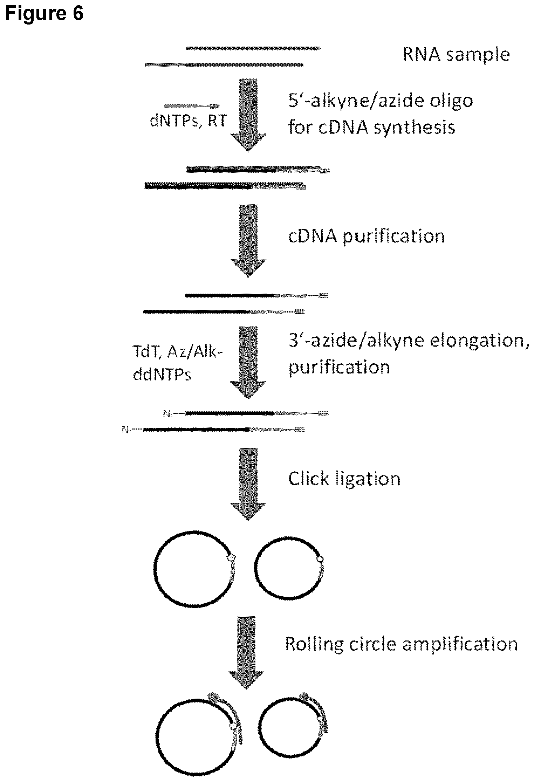

A variation of the amplification/sequencing method which can be used in the context of step i) of the inventive method for producing an mRNA tumor vaccine is shown in Figure 6. In such alternative method, as the first primers, various different alternatives and respective polynucleotides can be considered. Firstly, primers modified with a first partner of a pair of azide and alkyne molecules at the 5'-end can be prepared by known solid-phase methods and used for reverse transcription as described above in steps a) and b). After purification in step c) and addition of ddNTP, which is modified to carry the second partner of the pair of azide and alkyne molecules, via a template-independent polymerase in step d), cDNAs containing an azide and an alkyne residue at their ends would be generated. After removal of excess nucleotide, click ligation could be performed to generate circular single stranded cDNAs under click conditions (e.g. V. Cassinelli, et al. Angew. Chem. Int. Ed., 2015, 54, 7795-7798). In order to prevent concatenation, the double modified cDNAs are diluted before click ligation as described before (R. Kumar et al., J. Am. Chem. Soc., 2007, 129 (21), 6859-6864). Then, an alternative second primer which is complementary to at least a part of the first primer could be used instead of the second primer of step g) in order to amplify or sequence the template in a rolling circle amplification (BGI genomics NGS method). In this alternative method, accordingly, while most aspects described for the first inventive method still apply, addition of an adapter molecule described in step f) of the first aspect of the invention, is not applicable or required anymore. The alternative second primer, however, would also contain at its 3'-end a nucleotide which is complementary to the dideoxy nucleotide at the 3' end of the cDNA (before circularization via click reaction).

Instead of performing an amplification via steps h), it is also possible to directly perform sequencing according to known methods in step h'). E.g. a Sanger sequencing can be performed directly using the product obtained in step g) of the described method or the circular DNA/primer construct as shown at the bottom of Fig. 4 for the alternative method. Also single nucleotide sequencing methods can be applied using a product obtained by chain extension of the product of step g) or of the circular DNA/primer construct of the alternative method.

While the above-explained amplification and sequencing process is preferably used within the context of identifying tumor-specific mRNA transcripts in step i) of the inventive vaccine production method, it is important to note that any other method of genetic analysis is also applicable within the scope of the invention.

After having identified tumor specific mRNA transcripts and consequently vaccine mRNA candidates in step i), it is a further preferred embodiment to produce in step ii) as well as use for *in vitro* transfection in step iii) a first set of synthetic mRNA molecules which comprises not only one, but rather a multitude of tumor specific mRNAs for further evaluation. It is preferred to include 5 or more, more preferably 50 or more different to more-specific mRNA molecules for further testing. Depending on the number of tumor-specific mutations that have been detected by the genetic analysis, even more than 100 different mRNA molecules can be included for the first selection step in which in step iii) immune cells are transfected and mRNA encoding peptides are detected which are presented by the MHC complex of the immune cells.

While peptides, proteins or portions thereof encoded by this first set of synthetic mRNA molecules can vary in size, it seems especially advantageous that such peptides of proteins have a length of 5 to 30 amino acids, preferably 6 to 15 amino acids and most preferably 9 to 11 amino acids. Using peptides or proteins having such lengths are considered to be more easily bound and presented by the MHC.

While it is in principle possible to transfect immune cells with mixtures of different synthetic mRNA molecules, it is also possible for specific testing to transfect cells with a single synthetic mRNA molecule per transfection test sample only. In such setup, a variety of separate tests is performed to determine the ability of the various single mRNA molecules to elicit an immune response and the extent thereof.

The immune cells which are used for transfection in step iii) of the inventive method are preferably antigen presenting cells (APC) and, especially preferably, dendritic cells, monocytes, macrophages or B cells. After determining the immune response elicited by the mRNAs which are included in the first set of synthetic mRNA molecules, a further set of synthetic mRNA molecules is produced in step v) for a second screening step. Such second set of synthetic mRNAs includes nucleic acids which encode peptides or proteins which have been presented by the MHC complexes of the immune cells as determined in step iv). This second set of synthetic mRNA molecules preferably comprises 2 to 15 different mRNA molecules.

These synthetic mRNA molecules are then used in step vi) in the *in vitro* T cell-priming assay. In an especially preferred embodiment of the present invention, for the *in vitro* priming assay, T cells obtained from the patient are used for determining the immune response elicited by the second set of mRNA molecules. Also in this step a mixture of mRNA molecules can be used for the T-cell priming, or, the test can be performed in different batches with only a single type of synthetic mRNA molecule per test sample.

The *in vitro* T cell priming assay leads to identification of at least one peptide, protein or part thereof, expression of which in the patient by application of a corresponding mRNA vaccine can be used as an effective and specific anti-tumor immunisation.

After having described the inventive method for producing an mRNA tumor vaccine, especially a personalized mRNA-tumor vaccine, in detail, further aspects of the present invention will be discussed. For these aspects, all previous explanations and definitions apply equally in their respective contexts. Thus, all the terms and subjects explained and defined above are applicable also in the following.

A second aspect of the present invention relates to mRNA molecules which encode within their ORF a peptide, protein or portion thereof, which is specifically expressed in a tumor of the patient and which peptide, protein or portion thereof upon expression in immune cells of said patient elicits an immune response directed against such tumor. Especially, the mRNA molecules of the present invention encode a peptide, protein or portion thereof which upon contact with T cells of said patient triggers and/or increases an immune response in said T cells against said tumor.

Within the context of the present invention, it is preferred that the modified mRNA molecules include at least one of an alkyne-or azide-modification in at least one nucleotide within at least one of the ORF, the 5'- UTR, the 3'-UTR and the poly (A) tail region. In further preferred embodiments, the alkyne-or azide-modification is present in at least one nucleotide in the ORF and the UTRs, the ORF, the UTRs and the poly(A) tail, or the poly(A) tail, only. As already explained above, in inventive mRNA molecules at least one of the four standard types of nucleotides (AMP, CMP, GMP, UMP) are partly or completely modified, especially modified by an ethynyl- or azido-group.

As also already explained above, it is possible and a preferred embodiment of the present invention that the mRNA molecule includes at least one nucleotide, which is alkyne-modified, and at least one nucleotide, which is azide-modified. The advantage obtained by such double modification lies in the fact that the mRNA molecule can be connected to two different functional molecules, detectable labels or a combination of a detectable label and a functional molecule. The resulting possibilities and advantages and a high flexibility in obtaining advantageous properties are apparent to the skilled person.

Within the context of the present invention, the inventive mRNA molecule include the at least one of the four standard types of nucleotides in modified form in appropriate amounts, i.e. in amounts that the desired functions and properties of the mRNA molecule are not negatively affected by the modification. It has been found that modifications can be present in relatively large amounts and the presence of the modified form of an amino acid compared to the non-modified form in a ratio of 1:100 to 10:1, preferably 1:10 to 10:1, further preferably 1:4 to 4:1 and also preferable 1:2 to 2:1 is considered appropriate. Preferably, a 1:1, 1:4 or 1:10 combination of modified to non-modified nucleotides is included in the mRNA of the invention.

In further preferred embodiments, the inventive mRNA molecule contains otherwise modified natural or artificial nucleotides, like, for example, soda uridine and/or *N*1'-methyl pseudouridine. Furthermore, as already explained above, inventive mRNA molecules can contain one or more of detectable labels and functional molecules introduced via a click-reaction of the modified mRNA with a correspondingly modified alkyne- or azide-containing detectable label or functional molecule. As for the nucleotide modification, also for the modification of detectable labels or functional groups, suitable alkyne and azide groups are known to the skilled person and the preferred examples of such groups are applicable as described above.

The reaction of an alkyne-modified nucleotide within the modified mRNA and an azide-containing label or functional molecule or the reaction of an azide-modified nucleotide within the modified mRNA and an alkyne-containing label or functional molecule is performed under commonly known conditions to conduct the click-reaction. The type and size of labels and functional molecules is not particularly restricted. Preferred functional molecules include those, which are cell- or tissue-specific ligands that mediate targeted uptake of the mRNA into immune cells in a very efficient manner in order to elicit a suitably efficient immune response. E.g., the functional molecules should allow to attach or anchor the mRNA onto the surface of immune cells. Such cell- or tissue-specific targeting can be achieved for example by using specific antibodies or antibody-fragments, peptides, sugar moieties, small molecules or fatty acid moieties as the functional molecules and cell- or tissue-specific ligands. Respective substances have been described for a large number of targeting application and are available to the skilled person. Some preferred and exemplary targeting molecules are antibodies or antibody fragments or receptor ligands, which target cell-specific receptors like, e.g., the epidermal growth factor receptor, folate which targets the folate receptor, apolipoproteins which target endogenous low-density lipoprotein receptors or arachidonic acid which targets the endogenous cannabinoid receptors. Also, the amino acid sequence RGD or similar sequences have been found to mediate cell adhesion and can also be considered as preferred ligands within the context of the present invention.

Other information regarding the mRNA molecules of the present invention has been provided within the context of the detailed disclosure of the inventive method for producing a specific mRNA vaccine. Accordingly, the mRNA molecules of the present invention or any mRNA molecules, which are obtained by the inventive method, can be used for medical applications and as a vaccine, preferably for the prevention and/or treatment of a tumor disease. In preferred embodiments of this invention, the medical application includes the use as a personalized medicine. In this context, a treatment can be curative of prophylactic. With regard to prophylaxis, a corresponding mRNA vaccine can be obtained and applied in cases, where, for example, a hereditary condition is known, which renders the patient especially endangered to develop a tumor disease or, in case of a prevention of a recurrence of a tumor disease, from which the patient suffered previously.

In the context of such medical application, in a further aspect the present invention relates to a pharmaceutical composition, which comprises an mRNA molecule as described above. In such pharmaceutical composition, pharmaceutically acceptable carriers or excipients can be included. Furthermore, it is possible to provide a pharmaceutical combination, which comprises the mRNA molecule together with at least one further substance, which further substance is preferable an adjuvant, a substance which modulates, preferably stimulates, the immune system, and/or an anti-tumor agent. As such anti-tumor agent, a chemotherapeutic agent and/or a biological anti-tumor agent, for example an anti-tumor antibody can be used. The pharmaceutical composition of the present invention as well as the pharmaceutical combination described above are disclosed and claimed for use in medicine, preferable for the prevention and/or treatment of a tumor disease. The inventive compositions and combinations are personalized tumor vaccines, which can specifically target a tumor in a patient and, thus, allow for an efficient treatment while minimizing negative side effects for the patient.

A further aspect of the present invention is a method for treating a cancer disease or inhibit growth and/or (re-)development of a tumor in a patient by producing an mRNA tumor vaccine, which is specific for said cancer or tumor of said patient, by
i) identifying by genetic analysis specific mRNA transcripts, which are produced in a tumor cell of said patient and/or tumor-specific peptides or proteins encoded by said tumor-specific mRNA transcripts,
ii) producing a first set of synthetic mRNA molecules corresponding to the specific mRNA transcripts and/or encoding the tumor-specific peptides or proteins identified in step i), or portions thereof;
iii) in vitro transfecting immune cells with said first set of synthetic mRNA molecules produced in step ii) encoding said tumor-specific peptides, proteins or portions thereof and expressing said tumor-specific peptides, proteins or portions thereof in said transfected immune cells;
iv) identifying tumor-specific peptides, proteins or portions thereof which are immunogenic, particularly which are presented by MHC (class I or class II) molecules of said immune cells;
v) producing a set of synthetic mRNA molecules encoding immunogenic tumor-specific peptides or proteins identified in step iv) or portions thereof or peptides or proteins which include such immunogenic tumor-specific peptides or proteins or portions thereof;
vi) in vitro priming T cells with said set of synthetic mRNA molecules produced in step v) encoding said immunogenic tumor-specific peptides, proteins or portions thereof;
vii) identifying at least one peptide, protein or part thereof which triggers and/or increases an immune response in said T-cells in step vi);
viii) producing at least one synthetic mRNA molecule encoding a peptide, protein or portion thereof identified in step vii) for use as a personalized mRNA tumor vaccine in said patient;
and administering an effective amount of said mRNA tumor vaccine to said patient.

All aspects of the inventive method have been discussed in detail above with regard to the inventive method for producing an mRNA tumor vaccine. Accordingly, the disclosure provided in this description in the context of the other aspects of the invention equally apply to the respective features of the inventive method for treating a cancer disease.

In the inventive method, an effective amount of the mRNA tumor vaccine is administered to the patient. Within the context of the present invention, administering the inventive mRNA vaccine results in a stimulation and/or activation of immune effector cells. During this process, the immune effector cells are antigen-specifically activated and/or stimulated. Consequently, also the amount of antigen-specific effector cells, especially T-cells, is increased. Administration of the inventive mRNA vaccine to the patient can be performed by various methods, especially by parenteral, intravenous, subcutaneous, intranodal, intralymphatic, intraperitoneal administration.

While the administered mRNA vaccine can be applied in "naked" form, it is in principle also possible to transfer the mRNA vaccine into immature antigen presenting cells of this patient and to re-administer the cells to the individual.

The present invention in its various aspects presents new options for obtaining highly effective and patient-specific tumor vaccines. The various screening steps included in the present invention, starting with the genetic analysis of the patient's transcriptome and including further steps, which select candidate mRNAs for the vaccine based on their ability to elicit an immune response in different cells of the immune system, ensure a maximum effectivity for inducing an immune response in the patient, which is directed against the cancer cells or the tumor. Thus, a treatment by the inventive mRNA vaccine can help to avoid stressful forms of cancer treatment as they are currently applied, e.g., chemotherapy or radiation treatment. As the inventive mRNA only targets tumor and cancer cells, the patient is not further burdened by treatment methods, which not only attack cancer cells, but also destroy healthy cells. While in certain cases it might still be desirable or necessary to apply a chemotherapeutic substance or a radiation treatment in combination with the inventive tumor vaccine, at least the intensity of these treatments can be reduced substantially in such combination treatment.

The invention will be further apparent from the enclosed Figures and Examples.
**Figure 1** schematically shows entry of an inventive mRNA into immune cells and the release of proteins encoded by the inventive mRNA as well as the presentation of epitopes of the released proteins by MHC class I molecules. The Figure further shows the endocytosis of released protein and the presentation of epitopes thereof on MHC class II.
**Figure 2** shows step by step the preparation of inventive mRNA by transcription in the presence of modified nucleotide, the click-labeling, in this case with dye, and the subsequent transfection of cells, the tracking of transfection and the translation into a protein or a peptide.
**Figure 3** displays exemplary click-ready 5'-ends of adapter oligonucleotides which can be used within the context of the invention.
**Figure 4** shows a schematic representation of a common prior art PCR amplification of RNA for library preparation. The RNA sample is fragmented using an ultrasonic device, the fragments are (randomly) primed and a first strand synthesis (cDNA synthesis) is performed using a reverse transcriptase. A second strand is generated incorporating uracil instead of dTTP and the double-stranded DNA fragments are purified and blunted. dA tailing is performed to increase the efficiency of the subsequent enzymatic adapter ligation step. Another clean-up step involving size selection is done and the U-containing strand is removed before PCR enrichment.
**Figure 5** shows a schematic representation of a preferred amplification and sequencing method including poly(dT) priming to amplify mRNAs and provide full-length mRNA libraries.
**Figure 6** shows a schematic representation of an alternative workflow for full-length mRNA sequencing. A 5'-alkyne/azide modified primer is used for reverse transcription and the resulting cDNA is purified. An azide/alkyne nucleotide is incorporated to the 3'-end by the terminal deoxynucleotidyl transferase (TdT) and after removal of excess nucleotide click ligation is performed to generate circular ssDNA. By applying an at least partly complementary primer to the initial primer, amplification or even sequencing of the circular ssDNA would be possible.

The following items, which summarize important aspects of the present invention, are provided to supplement the above description:
1. A method for producing an mRNA-tumor vaccine for a patient suffering from a tumor, which method comprises the following steps:
   i) identifying by genetic analysis specific mRNA transcripts, which are produced in a tumor cell of said patient and/or tumor-specific peptides or proteins encoded by said tumor-specific mRNA transcripts,
   ii) producing a first set of synthetic mRNA molecules corresponding to the specific mRNA transcripts and/or encoding the tumor-specific peptides or proteins identified in step i), or portions thereof;
   iii) *in vitro* transfecting immune cells with said first set of synthetic mRNA molecules produced in step ii) encoding said tumor-specific peptides, proteins or portions thereof and expressing said tumor-specific peptides, proteins or portions thereof in said transfected immune cells;
   iv) identifying tumor-specific peptides, proteins or portions thereof which are immunogenic, particularly which are presented by MHC (class I or class II) molecules of said immune cells;
   v) producing a set of synthetic mRNA molecules encoding immunogenic tumor-specific peptides or proteins identified in step iv) or portions thereof or peptides or proteins which include such immunogenic tumor-specific peptides or proteins or portions thereof;
   vi) *in vitro* priming T cells with said set of synthetic mRNA molecules produced in step v) encoding said immunogenic tumor-specific peptides, proteins or portions thereof;
   vii) identifying at least one peptide, protein or part thereof which triggers and/or increases an immune response in said T-cells in step vi); and
   viii) producing at least one synthetic mRNA molecule encoding a peptide, protein or portion thereof identified in step vii) for use as a personalized tumor vaccine in said patient.
2. The method according to item 1, wherein one or more of the synthetic mRNA molecules produced in steps ii), v) and vii) is a modified mRNA molecule which includes at least one of an alkyne, alkene , azide or tetrazine modification in at least one nucleotide within at least one of the ORF, the 5'-UTR, the 3'-UTR and the poly(A) tail region. In addition to the preferred azide-/alkyne- modifications, alkene-/tetrazine-modifications may be also employed.
3. The method according to items 1 or 2, wherein the at least one synthetic mRNA molecule produced in step vii) is a modified mRNA molecule which includes at least one of an alkyne, alkene, azide or tetrazine modification in at least one nucleotide within at least one of the ORF, the 5'-UTR, the 3'-UTR and the poly(A) tail region.
4. The method according to anyone of items 1 to 3, wherein a modified mRNA molecule contains at least one modified nucleotide in
   a) the ORF and the UTRs,
   b) the ORF, the UTRs and the poly(A) tail, or
   c) only the poly(A) tail.
5. The method according to anyone of items 1 to 4, wherein in a modified mRNA molecule at least one of the four standard types of nucleotides (AMP, CMP, GMP, UMP) is partly or completely modified, preferably ethynyl-, cyclooctynyl-, trans-cyclooctenyl-, tetrazine- or azido-modified at uracil or adenine.
6. The method according to anyone of items 1 to 5, wherein in a modified mRNA molecule at least one nucleotide is alkyne-modified and at least one nucleotide is azide-modified.
7. The method according to anyone of items 1 to 6, wherein in a modified mRNA molecule at least one of the four standard types of nucleotides is present in modified form compared to the non-modified form in a ratio of 1:100 to 10:1, preferably 1:10 to 1:10, 1:4 to 4:1, or 1:1.
8. The method according to anyone of items 1 to 7, wherein a modified mRNA molecule contains otherwise modified natural or artificial nucleotides, preferably pseudouridine or *N*1-methylpseudouridine.
9. The method according to anyone of items 1 to 8, wherein a modified mRNA molecule contains one or more of a detectable label and a functional molecule introduced via a click reaction of the modified mRNA molecule with a correspondingly modified alkyne- or azide-containing detectable label or functional molecule.
10. The method according to item 9, wherein the detectable label is a colored or fluorogenic molecule and/or the functional molecule is a tissue or cell specific targeting group or ligand, preferably a sugar moiety, a (small) peptide, small molecule or a fatty acid moiety, or conjugates of different small molecules and natural oligomers.
11. The method according to anyone of items 1 to 10,, wherein the genetic analysis in step i) comprises sequencing of mRNA contained in a tumor-specific sample obtained from the patient and transcriptome analysis to detect tumor specific mRNA molecules.
12. The method according to item 11, wherein for the genetic analysis a full-length mRNA library of the tissue sample is prepared.
13. The method according to items 11 or 12, wherein the method comprises:
   a) providing at least one first primer, which first primer includes a sequence which is complementary to a sequence which is located at or near the 3'-end of the at least one RNA to be amplified, and adding said at least one first primer to the sample under conditions which allow for hybridization of at least one first primer to at least one RNA,
   b) reverse transcribing the at least one RNA under conditions including addition of a reverse transcriptase and nucleotides to the sample to provide full length cDNA(s) of the at least one RNA,
   c) purifying the sample at least from excess nucleotides
   d) adding a dideoxy nucleotide, which dideoxy nucleotide is modified at the 3'-position to include a first partner of a pair of azide and alkyne molecules, and a template-independent polymerase to attach a single 3'-azide- or 3'-alkyne modified dideoxy nucleotide at the 3'-end of the cDNA(s) obtained in step b),
   e) purifying the sample at least from excess modified dideoxy nucleotide added in step d),
   f) adding an adapter molecule, the adapter molecule comprising a polynucleotide sequence and, attached to its 5' end, a second partner of said pair of azide and alkyne molecules, under conditions to perform a click reaction and to ligate the adapter molecule to the 3'-modified cDNA(s) obtained in step d) under formation of a triazole linkage,
   g) adding a second primer, the second primer comprising a nucleotide sequence which is complementary to at least 6 of the nucleotides at the 5'-end of the adapter molecule and contains at its 3'-end a nucleotide which is complementary to the dideoxy nucleotide at the 3'-end of the cDNA(s), to effect hybridization and binding of the second primer to the ligated adapter/cDNA molecule obtained in step e) at a position overlapping the triazole linkage,
      and alternatively
   h) adding a DNA polymerase to achieve chain extension of the second primer to produce second strand DNA(s) including sequences complementary to the whole cDNA(s) obtained in step b), and either
   i) sequencing the second strand DNA(s) of step h), or
   j) adding a third primer which is identical to at least a part of the first primer, and amplifying the full-length cDNA(s) to obtain a full-length RNA library,
      or
   h') adding a DNA polymerase to achieve chain extension of the second primer and simultaneously determining the sequence of the second strand DNA(s) and the whole cDNA(s).
14. The method according to items 11 or 12, the method comprising:
   a) providing at least one first primer, which first primer includes a sequence which is complementary to a sequence which is located at or near the 3'-end of the at least one mRNA to be amplified, and which primer contains a modification by a first partner of a pair of azide and alkyne molecules at its 5' end, and adding said at least one first primer to the sample under conditions which allow for hybridization of at least one first primer to at least one mRNA,
   b) reverse transcribing the at least one mRNA under conditions including addition of a reverse transcriptase and nucleotides to the sample to provide full length cDNA(s) of the at least one mRNA,
   c) purifying the sample at least from excess nucleotides
   d) adding a dideoxy nucleotide, which dideoxy nucleotide is modified at the 3'-position to include a second partner of a pair of azide and alkyne molecules, and a template-independent polymerase to attach a single 3'-azide- or 3'-alkyne modified dideoxy nucleotide at the 3'-end of the cDNA(s) obtained in step b),
   e) purifying the sample at least from excess modified dideoxy nucleotide added in step d),
   f) performing a click ligation reaction to generate circular single stranded cDNA(s) under formation of a triazole linkage,
   g) adding a second primer, the second primer comprising a nucleotide sequence which is complementary to at least 6 of the nucleotides at the 5'-end of first primer and contains at its 3'-end a nucleotide which is complementary to the dideoxy nucleotide at the 3'-end of the cDNAs, to effect hybridization and binding of the second primer to the ligated circular first primer/cDNA molecule(s) obtained in step f) at a position overlapping the triazole linkage, and either
   h) adding a DNA polymerase to achieve chain extension of the second primer and subsequently amplify the full-length cDNA(s) or
   h') adding a DNA polymerase to achieve chain extension of the second primer and simultaneously determining the sequence of the circular single stranded DNA(s) including the whole cDNA(s).
15. The method according to anyone of items 1 to 1, wherein the first set of synthetic mRNA molecules produced in step ii) and used for *in vitro* transfection in step iii) comprises 2 or more, preferably 50 or more different tumor-specific mRNA molecules.
16. The method according to anyone of items 1 to 15, wherein the synthetic mRNA molecules produced in step ii) encode peptides, proteins or portions thereof having a length of 5to 30 amino acids, preferably 6 to 15 amino acids and most preferably 9 to 11 amino acids.
17. The method according to anyone of items 1 to 16, wherein in step iii) the immune cells are transfected with mixtures of different synthetic mRNA molecules or with single synthetic mRNA molecules.
18. The method according to anyone of items 1 to 17, wherein in step iii) of item 1 antigen presenting cells (APC) are transfected, preferably dendritic cells, macrophages and/or B cells.
19. The method according to anyone of items 1 to 18, wherein the second set of synthetic mRNA molecules produced in step v) of item 1 comprises 5 to 15 different mRNA molecules.
20. The method according to anyone of items 1 to 19, wherein in step vi), T-cells obtained from the patient are used for *in vitro* priming.
21. The method according to anyone of items 1 to 20, wherein the mRNA molecule produced in step viii) of item 1 is for use as a personalized tumor vaccine either alone or combined with at least one further substance, wherein the further substance is an adjuvant, a substance, which modulates, preferably stimulates the immune system, and/or an anti-tumor agent, e.g. a chemotherapeutic agent and/ or a biological anti-tumor agent such as an anti-tumor antibody.
22. An mRNA molecule, which encodes within its ORF a peptide, protein or portion thereof, which is specifically expressed in a tumor of a patient and which peptide, protein or portion thereof upon expression in immune cells of said patient elicits an immune response directed against such tumor.
23. The mRNA molecule according to item 22, which encodes a peptide, protein or portion thereof, which upon contact with T cells of said patient triggers and/or increases an immune response in said T-cells against said tumor
24. The mRNA molecule according to item 22 or 23, which is a modified mRNA molecule including at least one of an alkyne- or azide modification in at least one nucleotide within at least one of the ORF, the 5'-UTR, the 3'-UTR and the poly(a) tail region.
25. The mRNA molecule according to anyone of item 24 to 25, which includes at least one modified nucleotide in
   a) the ORF and the UTRs,
   b) the ORF, the UTRs and the poly(A) tail, or
   c) only the poly(A) tail.
26. The mRNA molecule according to anyone of items 24 to 25, wherein at least one of the four standard types of nucleotides (AMP, CMP, GMP, UMP) are partly or completely modified, e.g. ethynyl-, ethenyl-, tetrazine-or azido-modified at uracil or adenine, preferably ethynyl- or azido-modified.
27. The mRNA molecule according to anyone of items 24 to 26, wherein at least one nucleotide is alkyne-modified and at least one nucleotide is azide-modified.
28. The mRNA molecule according to anyone of items 24 to 27, wherein at least one of the four standard types of nucleotides is present in modified form compared to the non-modified form in a ratio of 1:100 to 10:1, preferably 1:10 to 10 to 1, 1:4 to 4:1 or 1:1.
29. The mRNA molecule according to anyone of items 24 to 28, which further contains otherwise modified natural or artificial nucleotides, preferably pseudouridine and/or *N*1-methylpseudouridine.
30. The mRNA molecule according to anyone of items 24 to 29, which contains one or more of a detectable label and a functional molecule introduced via a click reaction of the modified mRNA with a correspondingly modified alkyne-, alkene-, azide- or tetrazine-containing detectable label or functional molecule.
31. The mRNA molecule according to item 30, wherein the detectable label is a colored or fluorogenic molecule and/or the functional molecule is a tissue or cell specific targeting group or ligand, preferably a sugar moiety or a fatty acid moiety.
32 The mRNA molecule according to anyone of items 22 to 31, which encodes a peptide having a length of 5to 30 amino acids, preferably 6 to 15 amino acids and most preferably 9 to 11 amino acids.
33. The mRNA molecule according to anyone of items 22 to 32, or an mRNA molecule obtained by the method according to anyone of items 1 to 21 for use in medicine, preferably for the prevention and/or treatment of a tumor disease.
34. The mRNA molecule according to anyone of items 22 to 32 or an mRNA molecule obtained by the method according to anyone of items 1 to 21 for use as a personalized tumor vaccine.
35. The mRNA molecule according to anyone of items 22 to 32 for use according to item 33 or 34, wherein the treatment is curative or prophylactic, especially for preventing recurrence of a tumor disease.
36. A pharmaceutical composition comprising an mRNA molecule according to anyone of items 22 to 32 together with a pharmaceutically acceptable carrier or excipient.
37. A pharmaceutical combination comprising an mRNA molecule according to anyone of items 22 to 32 together with at least one further substance.
38. The pharmaceutical combination according to item 37, wherein the further substance is an adjuvant, a substance, which modulates, preferably stimulates the immune system, and/or an anti-tumor agent, e.g. a chemotherapeutic agent and/ or a biological anti-tumor agent such as an anti-tumor antibody.
39. The pharmaceutical composition according to item 36 or a pharmaceutical combination according to items 37 or 38 for use in medicine, preferably for the prevention and/or treatment of a tumor disease.
40. The pharmaceutical composition according to item 36 or a pharmaceutical combination according to items 37 or 38 for use as a personalized tumor vaccine.
41. The pharmaceutical composition according to items 36 or a pharmaceutical combination according to items 37 or 38 for use according to items 40 or 41, wherein the treatment is curative or prophylactic, especially for preventing recurrence of a tumor disease.
42. Method for treating a cancer disease or inhibit growth and/or (re-)development of a tumor in a patient by producing an mRNA tumor vaccine, which is specific for said cancer or tumor of said patient, by
   i) identifying by genetic analysis specific mRNA transcripts, which are produced in a tumor cell of said patient and/or tumor-specific peptides or proteins encoded by said tumor-specific mRNA transcripts,
   ii) producing a first set of synthetic mRNA molecules corresponding to the specific mRNA transcripts and/or encoding the tumor-specific peptides or proteins identified in step i), or portions thereof;
   iii) in vitro transfecting immune cells with said first set of synthetic mRNA molecules produced in step ii) encoding said tumor-specific peptides, proteins or portions thereof and expressing said tumor-specific peptides, proteins or portions thereof in said transfected immune cells;
   iv) identifying tumor-specific peptides, proteins or portions thereof which are immunogenic, particularly which are presented by MHC (class I or class II) molecules of said immune cells;
   v) producing a set of synthetic mRNA molecules encoding immunogenic tumor-specific peptides or proteins identified in step iv) or portions thereof or peptides or proteins which include such immunogenic tumor-specific peptides or proteins or portions thereof;
   vi) *in vitro* priming T cells with said set of synthetic mRNA molecules produced in step v) encoding said immunogenic tumor-specific peptides, proteins or portions thereof;
   vii) identifying at least one peptide, protein or part thereof which triggers and/or increases an immune response in said T-cells in step vi);
   viii) producing at least one synthetic mRNA molecule encoding a peptide, protein or portion thereof identified in step vii) for use as a personalized mRNA tumor vaccine in said patient;
   and administering an effective amount of said mRNA tumor vaccine to said patient.

## Claims

1. A method for producing an mRNA-tumor vaccine for a patient suffering from a tumor, which method comprises the following steps:
i) identifying by genetic analysis specific mRNA transcripts, which are produced in a tumor cell of said patient and/or tumor-specific peptides or proteins encoded by said tumor-specific mRNA transcripts,
ii) producing a first set of synthetic mRNA molecules corresponding to the specific mRNA transcripts and/or encoding the tumor-specific peptides or proteins identified in step i), or portions thereof;
iii) *in vitro* transfecting immune cells with said first set of synthetic mRNA molecules produced in step ii) encoding said tumor-specific peptides, proteins or portions thereof and expressing said tumor-specific peptides, proteins or portions thereof in said transfected immune cells;
iv) identifying tumor-specific peptides, proteins or portions thereof which are immunogenic, particularly which are presented by MHC (class I or class II) molecules of said immune cells;
v) producing a set of synthetic mRNA molecules encoding immunogenic tumor-specific peptides or proteins identified in step iv) or portions thereof or peptides or proteins which include such immunogenic tumor-specific peptides or proteins or portions thereof;
vi) *in vitro* priming T cells with said set of synthetic mRNA molecules produced in step v) encoding said immunogenic tumor-specific peptides, proteins or portions thereof;
vii) identifying at least one peptide, protein or part thereof which triggers and/or increases an immune response in said T-cells in step vi); and
viii) producing at least one synthetic mRNA molecule encoding a peptide, protein or portion thereof identified in step vii) for use as a personalized tumor vaccine in said patient.

2. The method according to claim 1, wherein one or more of the synthetic mRNA molecules produced in steps ii), v) and vii) is a modified mRNA molecule which includes at least one of an alkyne, alkene, azide or tetrazine modification in at least one nucleotide within at least one of the ORF, the 5'-UTR, the 3'-UTR and the poly(A) tail region, and/or wherein the at least one synthetic mRNA molecule produced in step vii) is a modified mRNA molecule which includes at least one of an alkyne, alkene, azide or tetrazine modification in at least one nucleotide within at least one of the ORF, the 5'-UTR, the 3'-UTR and the poly(A) tail region.

3. The method according to anyone of claims 1 and 2, wherein a modified mRNA molecule contains at least one modified nucleotide in
a) the ORF and the UTRs,
b) the ORF, the UTRs and the poly(A) tail, or
c) only the poly(A) tail.

4. The method according to anyone of claims 1 to 3, wherein in a modified mRNA molecule at least one of the four standard types of nucleotides (AMP, CMP, GMP, UMP) is partly or completely modified, preferably ethynyl-, cyclooctynyl- or azido-modified at uracil or adenine and/or wherein in a modified mRNA molecule at least one nucleotide is alkyne-modified and at least one nucleotide is azide-modified.

5. The method according to anyone of claims 1 to 4, wherein in a modified mRNA molecule at least one of the four standard types of nucleotides is present in modified form compared to the non-modified form in a ratio of 1:100 to 10:1, preferably 1:10 to 10:1, 1:4 to 4:1, or 1:1.

6. The method according to anyone of claims 1 to 5, wherein a modified mRNA molecule contains one or more of a detectable label and a functional molecule introduced via a click reaction of the modified mRNA molecule with a correspondingly modified alkyne-, alkene-, azide- or tetrazine-containing detectable label or functional molecule.

7. The method according to claim 9, wherein the detectable label is a colored or fluorogenic molecule and/or the functional molecule is a tissue or cell specific targeting group or ligand, preferably a sugar moiety, a small peptide, small molecule or conjugates of different small molecules and natural oligomers, or a fatty acid moiety

8. The method according to anyone of claims 1 to 7 , wherein the genetic analysis in step i) comprises sequencing of mRNA contained in a tumor-specific sample obtained from the patient and transcriptome analysis to detect tumor specific mRNA molecules, and/or wherein for the genetic analysis a full-length mRNA library of the tissue sample is prepared.

9. The method according to claim 8, wherein the method comprises:
a) providing at least one first primer, which first primer includes a sequence which is complementary to a sequence which is located at or near the 3'-end of the at least one RNA to be amplified, and adding said at least one first primer to the sample under conditions which allow for hybridization of at least one first primer to at least one RNA,
b) reverse transcribing the at least one RNA under conditions including addition of a reverse transcriptase and nucleotides to the sample to provide full length cDNA(s) of the at least one RNA,
c) purifying the sample at least from excess nucleotides
d) adding a dideoxy nucleotide, which dideoxy nucleotide is modified at the 3'-position to include a first partner of a pair of azide and alkyne molecules, and a template-independent polymerase to attach a single 3'-azide- or 3'-alkyne modified dideoxy nucleotide at the 3'-end of the cDNA(s) obtained in step b),
e) purifying the sample at least from excess modified dideoxy nucleotide added in step d),
f) adding an adapter molecule, the adapter molecule comprising a polynucleotide sequence and, attached to its 5' end, a second partner of said pair of azide and alkyne molecules, under conditions to perform a click reaction and to ligate the adapter molecule to the 3'-modified cDNA(s) obtained in step d) under formation of a triazole linkage,
g) adding a second primer, the second primer comprising a nucleotide sequence which is complementary to at least 6 of the nucleotides at the 5'-end of the adapter molecule and contains at its 3'-end a nucleotide which is complementary to the dideoxy nucleotide at the 3'-end of the cDNA(s), to effect hybridization and binding of the second primer to the ligated adapter/cDNA molecule obtained in step e) at a position overlapping the triazole linkage,
and alternatively
h) adding a DNA polymerase to achieve chain extension of the second primer to produce second strand DNA(s) including sequences complementary to the whole cDNA(s) obtained in step b), and either
i) sequencing the second strand DNA(s) of step h), or
j) adding a third primer which is identical to at least a part of the first primer, and amplifying the full-length cDNA(s) to obtain a full-length RNA library,
or
h') adding a DNA polymerase to achieve chain extension of the second primer and simultaneously determining the sequence of the second strand DNA(s) and the whole cDNA(s);
or
wherein the method comprises:
a) providing at least one first primer, which first primer includes a sequence which is complementary to a sequence which is located at or near the 3'-end of the at least one mRNA to be amplified, and which primer contains a modification by a first partner of a pair of azide and alkyne molecules at its 5' end, and adding said at least one first primer to the sample under conditions which allow for hybridization of at least one first primer to at least one mRNA,
b) reverse transcribing the at least one mRNA under conditions including addition of a reverse transcriptase and nucleotides to the sample to provide full length cDNA(s) of the at least one mRNA,
c) purifying the sample at least from excess nucleotides
d) adding a dideoxy nucleotide, which dideoxy nucleotide is modified at the 3'-position to include a second partner of a pair of azide and alkyne molecules, and a template-independent polymerase to attach a single 3'-azide- or 3'-alkyne modified dideoxy nucleotide at the 3'-end of the cDNA(s) obtained in step b),
e) purifying the sample at least from excess modified dideoxy nucleotide added in step d),
f) performing a click ligation reaction to generate circular single stranded cDNA(s) under formation of a triazole linkage,
g) adding a second primer, the second primer comprising a nucleotide sequence which is complementary to at least 6 of the nucleotides at the 5'-end of first primer and contains at its 3'-end a nucleotide which is complementary to the dideoxy nucleotide at the 3'-end of the cDNAs, to effect hybridization and binding of the second primer to the ligated circular first primer/cDNA molecule(s) obtained in step f) at a position overlapping the triazole linkage, and either
h) adding a DNA polymerase to achieve chain extension of the second primer and subsequently amplify the full-length cDNA(s) or
h') adding a DNA polymerase to achieve chain extension of the second primer and simultaneously determining the sequence of the circular single stranded DNA(s) including the whole cDNA(s).

10. The method according to anyone of claims 1 to 9, wherein the first set of synthetic mRNA molecules produced in step ii) and used for *in vitro* transfection in step iii) comprises 2 or more, preferably 50 or more different tumor-specific mRNA molecules, and/or wherein the synthetic mRNA molecules produced in step ii) encode peptides, proteins or portions thereof having a length of 5t o 30 amino acids, preferably 6 to 15 amino acids and most preferably 9 to 11 amino acids.

11. The method according to anyone of claims 1 to 10, wherein in step iii) the immune cells are transfected with mixtures of different synthetic mRNA molecules or with single synthetic mRNA molecules, preferably wherein the immune cells are antigen presenting cells (APC), more preferably dendritic cells, macrophages and/or B cells.

12. The method according to anyone of claims 1 to 11, wherein the second set of synthetic mRNA molecules produced in step v) comprises 2 to 15 different mRNA molecules.

13. The method according to anyone of claims 1 to 12, wherein in step vi), T-cells obtained from the patient are used for *in vitro* priming.

14. The method according to anyone of claims 1 to 20, wherein the mRNA molecule produced in step viii) of claim 1 is for use as a personalized tumor vaccine either alone or combined with at least one further substance, wherein the further substance is an adjuvant, a substance, which modulates, preferably stimulates the immune system, and/or an anti-tumor agent, e.g. a chemotherapeutic agent and/ or a biological anti-tumor agent such as an anti-tumor antibody.

15. An mRNA molecule, which encodes within its ORF a peptide, protein or portion thereof, which is specifically expressed in a tumor of a patient and which peptide, protein or portion thereof upon expression in immune cells of said patient elicits an immune response directed against such tumor, and/or an mRNA molecule, which encodes a peptide, protein or portion thereof, which upon contact with T cells of said patient triggers and/or increases an immune response in said T-cells against said tumor,
preferably wherein the mRNA encodes a peptide having a length of 5 to 30 amino acids, preferably 6 to 15 amino acids, and most preferably 9 to 11 amino acids.

16. The mRNA molecule according to claim 15, which is a modified mRNA molecule including at least one of an alkyne-, alkene, azide or tetrazine modification in at least one nucleotide within at least one of the ORF, the 5'-UTR, the 3'-UTR and the poly(a) tail region; especially which includes at least one correspondingly modified nucleotide in
a) the ORF and the UTRs,
b) the ORF, the UTRs and the poly(A) tail, or
c) only the poly(A) tail,
and optionally
wherein at least one of the four standard types of nucleotides (AMP, CMP, GMP, UMP) are partly or completely modified, preferably ethynyl- or azido-modified at uracil or adenine, and/or
wherein at least one nucleotide is alkyne-modified and at least one nucleotide is azide-modified, and/or
wherein at least one of the four standard types of nucleotides is present in modified form compared to the non-modified form in a ratio of 1:100 to 10:1, preferably 1:10 to 10:1, 1:4 or 4:1, or 1:1, and/or
wherein the mRNA which contains one or more of a detectable label and a functional molecule introduced via a click reaction of the modified mRNA with a correspondingly modified alkyne-, alkene-, azide- or tetrazine-containing detectable label or functional molecule, preferably wherein the detectable label is a colored or fluorogenic molecule and/or the functional molecule is a tissue or cell specific targeting group or ligand, preferably a sugar moiety or a fatty acid moiety.

17. A pharmaceutical composition comprising an mRNA molecule according to anyone of claims 15 or 16 together with a pharmaceutically acceptable carrier or excipient, and optionally together with at least one further substance, preferably wherein the further substance is an adjuvant, a substance, which modulates, preferably stimulates the immune system, and/or an anti-tumor agent, e.g. a chemotherapeutic agent and/ or a biological anti-tumor agent such as an anti-tumor antibody.

18. The mRNA molecule according to anyone of claims 15 or 16, or an mRNA molecule obtained by the method according to anyone of claims 1 to 14, or the pharmaceutical composition according to claim 17 for use in medicine, preferably for the prevention and/or treatment of a tumor disease, and/or for use as a personalized tumor vaccine, and/or wherein the treatment is curative or prophylactic, especially for preventing recurrence of a tumor disease.

19. Method for treating a cancer disease or inhibit growth and/or (re-)development of a tumor in a patient by producing an mRNA tumor vaccine, which is specific for said cancer or tumor of said patient, by
i) identifying, preferably by genetic analysis, specific mRNA transcripts, which are produced in a tumor cell of said patient and/or tumor-specific peptides or proteins encoded by said tumor-specific mRNA transcripts,
ii) producing a first set of synthetic mRNA molecules corresponding to the specific mRNA transcripts and/or encoding the tumor-specific peptides or proteins identified in step i), or portions thereof;
iii) in vitro transfecting immune cells with said first set of synthetic mRNA molecules produced in step ii) encoding said tumor-specific peptides, proteins or portions thereof and expressing said tumor-specific peptides, proteins or portions thereof in said transfected immune cells;
iv) identifying tumor-specific peptides, proteins or portions thereof which are immunogenic, particularly which are presented by MHC (class I or class II) molecules of said immune cells;
v) producing a set of synthetic mRNA molecules encoding immunogenic tumor-specific peptides or proteins identified in step iv) or portions thereof or peptides or proteins which include such immunogenic tumor-specific peptides or proteins or portions thereof;
vi) *in vitro* priming T cells with said set of synthetic mRNA molecules produced in step v) encoding said immunogenic tumor-specific peptides, proteins or portions thereof;
vii) identifying at least one peptide, protein or part thereof which triggers and/or increases an immune response in said T-cells in step vi);
viii) producing at least one synthetic mRNA molecule encoding a peptide, protein or portion thereof identified in step vii) for use as a personalized mRNA tumor vaccine in said patient;
and administering an effective amount of said mRNA tumor vaccine to said patient.
